# EUROPEAN PATENT APPLICATION

(11) **EP 1 319 409 A1**
(43) Date of publication of application: **18.06.2003**
(21) Application number: 01967797.0
(22) Date of filing: 21.09.2001
(51) Int. Cl.: A61K 47/38, A61K 47/12, A61K 9/30, A61K 9/36, A61K 9/48, A61K 9/16, A61K 9/14, A61K 31/519, A61K 31/4365, C07D 495/04

(54) **SOLID PREPARATIONS**

(30) Priority: 22.09.2000 JP 2000000289
(71) Applicant: Takeda Chemical Industries, Ltd., Osaka-shi, Osaka 540-8645 (JP)
(72) Inventor: NAKANO, Yoshinori, Takarazuka-shi, Hyogo 665-0816 (JP); YONEYAMA, Shuji, Suita-shi, Osaka 565-0853 (JP); OCHI, Masashi, Amagasaki-shi, Hyogo 661-0044 (JP)
(74) Representative: von Kreisler, Alek, Dipl.-Chem.
(86) International application number: JP0108264
(87) International publication number: WO02024230

(57) **Abstract**

The present invention aims at providing a granule comprising a slightly soluble in water and highly water-repellent physiologically active substance in a large content, and a solid preparation comprising the granule, which is superior in disintegration property and dissolution of the physiologically active substance from the preparation. The present invention relates to (1) a granule comprising a physiologically active substance and a cellulose-type disintegrant, (2) a granule comprising a physiologically active substance, a cellulose-type disintegrant and a binder, (3) a solid preparation comprising the granule described in (1) or (2), a cellulose-type disintegrant and a stearic acid-type lubricant and (4) the solid preparation described in (3), which is an oval tablet.

## Description

### Technical Field

The present invention relates to a granule, a solid preparation and a production method thereof.

### Background Art

When a general binder solution obtained by dissolving or partially dispersing a binder in water is used for the granulating step of a preparation comprising a large content of a slightly water-soluble and a highly water-repellent physiologically active substance, granulation does not proceed, operationality in the steps up to the tableting step is poor and the content uniformity of the physiologically active substance in the uncoated tablets obtained by tablet-making may not be consistent.

Generally, moreover, the tablet is selected as the dosage form of a solid preparation, and the shape of the tablet employed, with consideration of easy administration and productivity, is round. When increased weight of the tablet is needed in view of the content and proportion of the physiologically active substance in the tablet, however, such tablet cannot be easily swallowed and shows delay of drug dissolution.

### Disclosure of the Invention

Granules uniformly comprising a large content of a slightly water-soluble and highly water-repellent physiologically active substance have not been obtained. In addition, a highly disintegrable solid preparation or a solid preparation comprising a large content of a physiologically active substance has not been obtained as yet from conventionally known granules.

The present inventors have intensively studied with the aim of solving the aforementioned problems and found that a granule showing superior operationality up to the tableting step and comprising a physiologically active substance uniformly in a large content; even if it is slightly water-soluble and(or) water-repellent, such as a physiologically active substance having a gonadotropin GnRH (Gonadotropin releasing hormone) antagonistic activity, and further a solid preparation comprising a physiologically active substance uniformly in a large content, can be obtained by adding a cellulose-type disintegrant such as calcium carboxymethylcellulose (hereinafter to be sometimes abbreviated as CMC-Ca) and the like); and particularly that a granule showing superior operationality up to the tableting step, a granule comprising a physiologically active substance uniformly in a large content and further a solid preparation comprising a physiologically active substance uniformly in a large content can be obtained by mixing a physiologically active substance with a cellulose-type disintegrant and a solution (or suspension) comprising a binder (e.g., hydroxymethylcellulose (hereinafter to be sometimes abbreviated as HPC) and the like.

Furthermore, they have found that a solid preparation comprising the granule can be a solid preparation superior in disintegration property. In addition, they have found that, by processing the solid preparation into a tablet, particularly an oval tablet, the dissolution property is improved unexpectedly.

They have also found that, when the physiologically active substance is unstable to light and changes its color due to light, the substance can be stably preserved for a long time even under non-shading conditions, by making a film-coated tablet.

Further studies of the above-mentioned points have resulted in the completion of the present invention.

The present invention relates to
(1) a granule comprising a physiologically active substance and a cellulose-type disintegrant;
(2) the granule described in the aforementioned (1), wherein the cellulose-type disintegrant is a compound selected from calcium carboxymethylcellulose, carboxymethylcellulose, croscarmellose sodium and low substituted hydroxypropyl cellulose or comprises two or more compounds therefrom in combination;
(3) the granule described in the aforementioned (1), wherein the cellulose-type disintegrant is calcium carboxymethylcellulose;
(4) the granule described in the aforementioned (1), wherein the physiologically active substance is slightly soluble in water;
(5) the granule described in the aforementioned (1), wherein the physiologically active substance is water-repellent;
(6) the granule described in the aforementioned (1), wherein the physiologically active substance is slightly soluble in water and water-repellent;
(7) the granule described in the aforementioned (1), wherein the physiologically active substance has a contact angle of not less than 80 degrees;
(8) the granule described in the aforementioned (1), wherein the physiologically active substance is a compound having a partial structure represented by the formula: 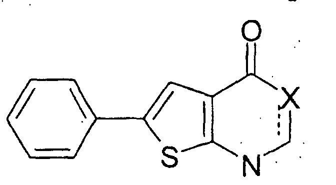 [wherein X represents a carbon atom or a nitrogen atom, and - - - represents a single bond or a double bond] or a salt thereof [hereinafter also abbreviated as "compound (X)"];
(9) the granule described in the aforementioned (8), wherein the compound (X) is a compound represented by the formula: 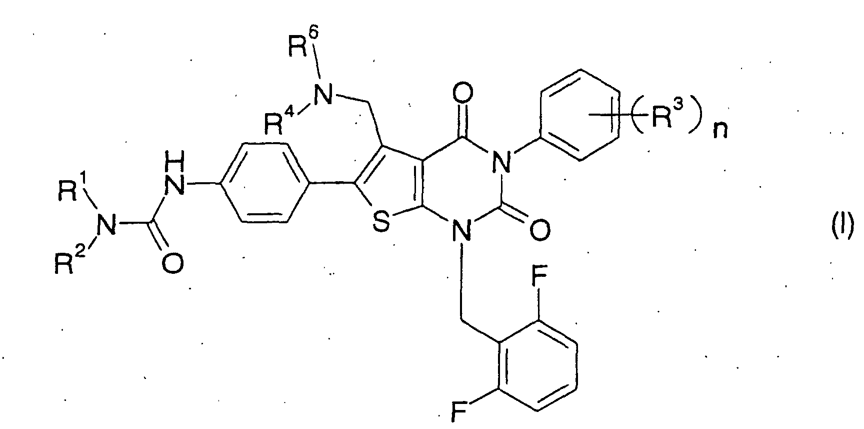 [wherein R¹ and R² each represent a hydrogen atom, a hydroxy group, a C₁₋₄ alkoxy group, a C₁₋₄ alkoxy-carbonyl group or a C₁₋₄ alkyl group which may have a substituent,
   R³ represents a hydrogen atom, a halogen atom, a hydroxy group or a C₁₋₄ alkoxy group which may have a substituent, or
   adjacent two R³s may be linked to form a C₁₋₄ alkylenedioxy group;
   R⁴ represents a hydrogen atom or a C₁₋₄ alkyl group;
   R⁶ represents a C₁₋₄ alkyl group which may have a substituent or a group represented by the formula: 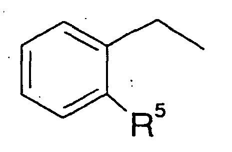 (wherein R⁵ represents a hydrogen atom or R⁴ and R⁵ may be linked to form a heterocycle); and
   n represents an integer of 0 to 5] or a salt thereof [hereinafter also abbreviated as "compound (I)"];
(10) the granule described in the aforementioned (8), wherein compound (X) is 5-(N-benzyl-N-methylaminomethyl)-1-(2,6-difluorobenzyl)-6-[4-(3-methoxyureido)phenyl]-3-phenylthieno [2,3-d]pyrimidine-2,4(1H,3H)-dione or a salt thereof;
(11) the granule described in the aforementioned (8), wherein compound (X) is a compound represented by the formula: 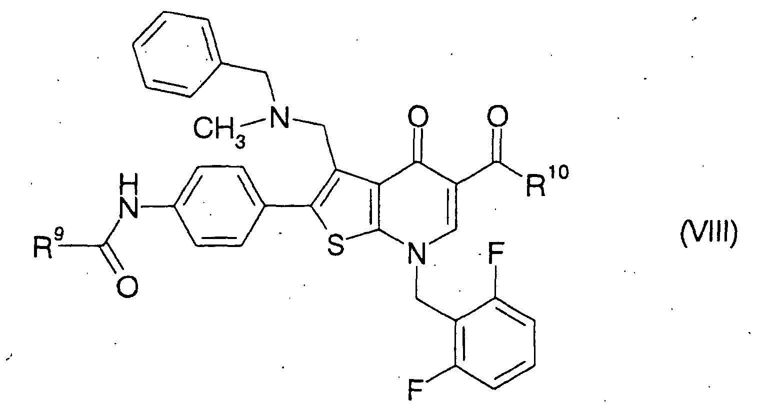 [wherein R⁹ represents an optionally substituted C₁₋₇ alkyl group, an optionally substituted C₃₋₇ cycloalkyl group, an optionally substituted C₁₋₆ alkoxyamino group or an optionally substituted hydroxyamino group, and
   R¹⁰ represents an optionally substituted C₁₋₇ alkyl group or an optionally substituted phenyl group, respectively; or
   when R⁹ is an unsubstituted C₁₋₇ alkyl group, R¹⁰ represents a substituted C₁₋₇ alkyl group or a substituted phenyl] or a salt thereof [hereinafter also abbreviated as "compound (VIII)"];
(12) the granule described in the aforementioned (8), wherein compound (X) is 3-(N-benzyl-N-methylaminomethyl)-4,7-dihydro-5-isobutyryl-7-(2,6-difluorobenzyl)-2-[4-[(1-hydroxycyclopropyl)carbonylamino]phenyl]-4-oxothieno[2,3-b]pyridine or a salt thereof;
(13) the granule described in the aforementioned (1), which is obtained by mixing and drying a physiologically active substance, a cellulose-type disintegrant and a solution comprising a binder;
(14) the granule described in the aforementioned (13), wherein the binder is a compound selected from hydroxypropyl cellulose, pre-gelatinized starch, sucrose, gelatin, powdered acacia, methylcellulose, hydroxypropyl methylcellulose, polyvinylpyrrolidone, dextrin and pullulan or two or more compounds thereof in combination;
(15) the granule described in the aforementioned (13), wherein the binder is hydroxypropyl cellulose;
(16) the granule described in the aforementioned (13), wherein the solution comprising a binder is an alcohol solution;
(17) the granule described in the aforementioned (16), wherein the alcohol has 1 to 3 carbon atoms;
(18) the granule described in the aforementioned (16), wherein the alcohol is ethanol;
(19) the granule described in the aforementioned (1), which is used for tableting;
(20) a production method of granule, which comprises mixing and drying a physiologically active substance, a cellulose-type disintegrant and a solution comprising a binder;
(21) a solid preparation comprising the granule described in the aforementioned (1) to (18);
(22) the solid preparation described in the aforementioned (21), comprising the granule described in the aforementioned aforementioned (1) to (18), a cellulose-type disintegrant and a stearic acid-type lubricant;
(23) the solid preparation described in the aforementioned (22), wherein the cellulose-type disintegrant is a compound selected from calcium carboxymethylcellulose, carboxymethylcellulose, croscarmellose sodium and low substituted hydroxypropyl cellulose or comprises two or more compounds therefrom in combination;
(24) the solid preparation described in the aforementioned (22), wherein the cellulose-type disintegrant is calcium carboxymethylcellulose;
(25) the solid preparation described in the aforementioned (22), wherein the stearic acid-type lubricant is magnesium stearate;
(26) the solid preparation described in the aforementioned (21), which is a film-coated tablet;
(27) the solid preparation described in the aforementioned (21), which is an oval tablet;
(28) the solid preparation described in the aforementioned (21), which is a capsule;
(29) the solid preparation described in the aforementioned (21), which is a granule or fine granule;
(30) a granule comprising compound (I);
(31) the granule described in the aforementioned (30), which comprises 5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione or a salt thereof;
(32) the granule described in the aforementioned (30), which is obtained by mixing and drying compound (I) and a solution comprising a binder;
(33) the granule described in the aforementioned (32), wherein the binder is a compound selected from hydroxypropyl cellulose, pre-gelatinized starch, sucrose, gelatin, powdered acacia, methylcellulose, hydroxypropyl methylcellulose, polyvinylpyrrolidone, dextrin and pullulan or comprises two or more compounds in combination;
(34) the granule described in the aforementioned (32), wherein the solution comprising a binder is an alcohol solution;
(35) the granule described in the aforementioned (34), wherein the alcohol has 1 to 3 carbon atoms;
(36) the granule described in the aforementioned (34), wherein the alcohol is ethanol;
(37) the granule described in the aforementioned (30), which is used for tableting;
(38) a production method of a granule, which comprises mixing and drying compound (I) and a solution comprising a binder,;
(39) a solid preparation comprising the granule described in the aforementioned (30) to (36);
(40) the solid preparation described in the aforementioned (39), which comprises the granule described in the aforementioned (30) to (36), a cellulose-type disintegrant and a stearic acid-type lubricant;
(41) the solid preparation described in the aforementioned (40), wherein the cellulose-type disintegrant is a compound selected from calcium carboxymethylcellulose, carboxymethylcellulose, croscarmellose sodium and low substituted hydroxypropyl cellulose or comprises two or more compounds thereof in combination;
(42) the solid preparation described in the aforementioned (40), wherein the cellulose-type disintegrant is calcium carboxymethylcellulose;
(43) the solid preparation described in the aforementioned (40), wherein the stearic acid-type lubricant is magnesium stearate;
(44) the solid preparation described in the aforementioned (39), which is a film-coated tablet;
(45) the solid preparation described in the aforementioned (39), which is an oval tablet;
(46) the solid preparation described in the aforementioned (39), which is a capsule;
(47) the solid preparation described in the aforementioned (39), which is a granule or a fine granule;
(48) the solid preparation described in the aforementioned (39), which is a gonadotropin releasing hormone antagonist;
(49) the solid preparation described in the aforementioned (39), which is an agent for the prophylaxis or treatment of sex hormone-dependent diseases and the like.

### Brief Description of the Drawing

The dissolution rate of each uncoated tablet obtained in Example 1-(3) and Example 6 is shown.

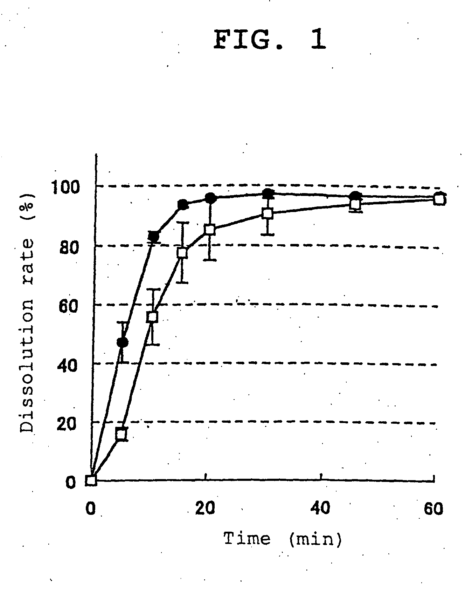

In Fig. 1, -•- shows a dissolution curve of compound A from an oval tablet and -□- shows a dissolution curve of compound A from a round tablet.

### Detailed Description of the Invention

Preferably, such "physiologically active substance" to be used in the present invention has a molecular weight of not more than about 1000, preferably not more than about 900, more preferably not more than about 800, and most preferably not more than about 700. In the present invention, the "physiologically active" substance which is slightly water-soluble and/or water-repellent markedly shows its effect.

Such "slightly water-soluble physiologically active substance" to be used in the present invention has a solubility of, for example, not more than 0.1% (w/v), preferably not more than 0.01% (w/v). The term "solubility" used herein means concentration of drug in a supernatant which is obtained by centrifugal separation of unsolved drug, after shaking a mixture prepared by adding the drug of which amount is greater than the solubility to the second solution defined in 14th revision Japanese Pharmacopoeia [0.2 M phosphate-buffer (pH approx. 6.8), for more than 30 minutes at room temperature (about 15 to about 25°C) at a rate of not less than 100 times per minutes using, for example, a Recipro shaker (model SR-I, Taiyo Scientific Industrial Co., Ltd.).

The "water-repellent physiologically active substance" to be used in the present invention is exemplified by a substance difficult to get wet, or having a large contact angle, and the like. A specific example thereof is a physiologically active substance having a contact angle of not less than 40 degrees, preferably not less than 60 degrees, more preferably not less than 80 degrees, as measured by forming a compression-molded product (300 mg) having a diameter of 13 mm with Shimadzu autograph (AG-5000B, manufactured by Shimadzu Corporation) at a load of 1 ton/cm², placing one drop of purified water on the surface thereof with a syringe, and determining the contact angles of the water drop at 15 seconds after placing with an automatic contact angle meter (CA-Z, manufactured by Kyowa Interface Science Co., Ltd.), and the like. The contact angle of a representative highly water-repellent substance, magnesium stearate, compound A to be mentioned later: (5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione), and compound B to be mentioned later: (3-(N-benzyl-N-methylaminomethyl)-4,7-dihydro-5-isobutyryl-7-(2,6-difluorobenzyl)-2-[4-[(1-hydroxycyclopropyl)carbonylamino]phenyl]-4-oxothieno[2,3-b]pyridine) as measured according to the aforementioned method were about 96 degrees, about 90 degrees and about 88 degrees respectively.

As the "physiologically active substance" to be used in the present invention, GnRH antagonist, GnRH agonist, antibiotic and the like are mentioned. Of these, GnRH antagonist is preferable.

As such "GnRH antagonist", any compounds are possible insofar as they have a GnRH antagonistic activity, and examples of which include compounds having a partial structure (basic structure) represented by the formula: 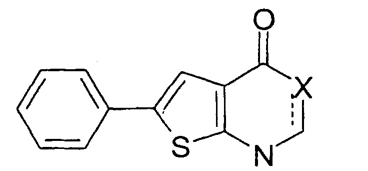 [wherein X represents a carbon atom or a nitrogen atom, - - - represents a single bond or a double bond] or salts thereof, and more specifically the aforementioned compound (I), compound (VIII), salts thereof and the like are recited.

Definitions for the respective substituents in the above formula (I) are as follows.

Examples of "C₁₋₄ alkoxy group" represented by R¹ or R² include methoxy, ethoxy, propoxy, isopropoxy, butoxy, tert-butoxy and the like. Among these, C₁₋₃ alkoxy group is preferred. Methoxy is more preferred.

Examples of "C₁₋₄ alkoxy-carbonyl group" represented by R¹ or R² include methoxycarbonyl, ethoxycarbonyl, propoxycarbonyl, isopropoxycarbonyl, butoxycarbonyl, tert-butoxycarbonyl and the like. Among these C₁₋₃ alkoxy-carbonyl group is preferred. Methoxycarbonyl is more preferred.

Examples of the "C₁₋₄ alkyl group" in the "C₁₋₄ alkyl group which may have a substituent" represented by R¹ or R² include linear C₁₋₄ alkyl groups (e.g., methyl, ethyl, propyl, butyl, etc.) and branched C₃₋₄ alkyl groups (e.g., isopropyl, isobutyl, sec-butyl, tert-butyl, etc.). Among these, C₁₋₃ alkyl groups are preferred. In particular, ethyl is preferred.

Examples of "substituent" in "C₁₋₄ alkyl group which may have a substituent" represented by R¹ or R² include (i) hydroxy, (ii) C₁₋₇ acyloxy (e.g., C₁₋₆ alkyl-carbonyloxy such as acetoxy and propionyloxy), (iii) benzoyloxy, (iv) amino groups which may have one or two substituent(s) selected from the group consisting of C₁₋₆ alkoxy-carbonyl (e.g., methoxycarbonyl, ethoxycarbonyl, tert-butoxycarbonyl, etc.), benzyloxycarbonyl, C₁₋₄ acyl (e.g., C₁₋₃ alkyl-carbonyl such as acetyl and propionyl), C₁₋₄ alkyl (e.g., methyl, ethyl, propyl, butyl, etc.), C₁₋₃ alkylsulfonyl (e.g., methanesulfonyl) and the like (for example, amino, dimethylamino, methoxycarbonylamino, ethoxycarbonylamino, tert-butoxycarbonylamino, benzyloxycarbonylamino, acetylamino, methanesulfonylamino and the like), (v) C₁₋₁₀ alkoxy (e.g., methoxy, ethoxy, propoxy, tert-butoxy, etc.), (vi) C₃₋₇ cycloalkyloxycarbonyloxy-C₁₋₃ alkoxy (e.g., cyclohexyloxycarbonyloxy-1-ethoxy, etc.) and (vii) C₁₋₃ alkoxy-C₁₋₃ alkoxy (e.g., methoxymethoxy, methoxyethoxy, etc.). Among these, hydroxy is preferred.

The "C₁₋₄ alkyl group" in the "C₁₋₄ alkyl group which may have a substituent" represented by R¹ or R² may have, for example, 1 to 5, preferably 1 to 3, substituent(s) mentioned above at positions where substitution is possible, and if the number of substituents is two or more, the respective substituents may be the same or different.

It is preferred that one of R¹ and R² is a hydrogen atom, while the other of R¹ and R² is C₁₋₃ alkoxy group.

Examples of the "halogen atom" represented by R³ include fluorine, chlorine, bromine and iodine. Among these, chlorine is preferred.

Examples of the "C₁₋₄ alkoxy group" in the "C₁₋₄ alkoxy group which may have a substituent" represented by R³ include methoxy, ethoxy, propoxy, isopropoxy, butoxy, tert-butoxy and the like. Among these, methoxy is preferred.

Examples of "substituent(s)" in "C₁₋₄ alkoxy group which may have a substituent" represented by R³ include those as same as recited for the "substituent" in "C₁₋₄ alkyl group which have a substituent" represented by R¹ or R². Among these, C₁₋₄ alkoxy group is preferred.

The C₁₋₄ alkoxy group may have, for example, 1 to 5, preferably 1 to 3, substituent(s) mentioned above at positions where substitution is possible, and if the number of substituents is two or more, the respective substituents may be the same or different.

Examples of "C₁₋₄ alkylenedioxy group" which is formed by linkage of adjacent two R³s include methylenedioxy, ethylenedioxy and the like.

Preferably, R³ is a hydrogen atom.

Examples of the "C₁₋₄ alkyl group" represented by R⁴ include linear C₁₋₄ alkyl groups (e.g., methyl, ethyl, propyl, butyl, etc.), branched C₃₋₄ alkyl groups (e.g., isopropyl, isobutyl, sec-butyl, tert-butyl, etc.) and the like. Among these, C₁₋₃ alkyl groups are preferred. In particular, methyl is preferred.

Examples of the "optionally substituted C₁₋₄ alkyl group which may have a substituent" represented by R⁶ include "C₁₋₄ alkyl groups" represented by R¹ or R².

Examples of the "heterocycle" formed by linkage of R⁴ and R⁵ include 5- or 6-membered nitrogen-containing heterocyclic groups. When R⁴ and R⁵ are linked to each other, examples of groups represented by the formula: 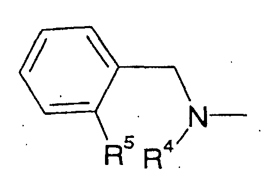 include the groups represented by the formulas 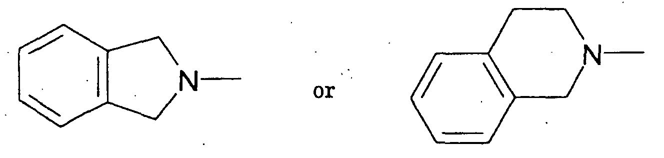 Among these, a group represented by the formula: 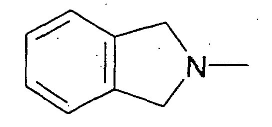 is preferred.

Preferably, R⁶ is a group represented by the following formula: 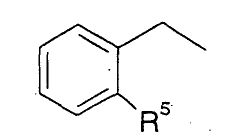 [wherein R⁵ has the same meaning as described above].

Preferably, R⁴ is a C₁₋₃ alkyl group and R⁵ is a hydrogen atom.

Preferably, n is an integer of 0 to 2.

Examples of preferred compounds in compound (I) include those wherein R¹ is hydroxy group, methoxy group or C₁₋₃ alkyl group; R² is a hydrogen atom or a C₁₋₃ alkyl group; R⁴ is C₁₋₃ alkyl group; R⁶ is a benzyl group; and n is 0, and salts thereof.

Among others, compounds wherein R¹ represents a methoxy group, R² and R⁵ each represent a hydrogen atom; R⁴ is a C₁₋₃ alkyl group; R⁶ is a benzyl group; and n is 0, and salts thereof are recited.

Concrete examples of compound (I) include 5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione,
5-(N-benzyl-N-methylaminomethyl)-1-(2,6-difluorobenzyl)-6-[4-(3-hydroxyureido)phenyl]-3-phenylthieno[2,3-d]pyrimidine-2,4(1H,3H)-dione,
5-(N-benzyl-N-methylaminomethyl)-1-(2,6-difluorobenzyl)-6-[4-(3-methylureido)phenyl]-3-phenylthieno[2,3-d]pyrimidine-2,4(1H,3H)-dione,
5-(N-benzyl-N-methylaminomethyl)-1-(2,6-difluorobenzyl)-6-[4-(3-ethylureido)phenyl]-3-phenylthieno[2,3-d]pyrimidine-2,4(1H,3H)-dion and salts thereof are exemplified.

Among them, 5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione or a salt thereof is preferred.

Definition of each substituent in the above formula (VIII) will be described below.

Examples of the "C₁₋₇ alkyl group" in the "optionally substituted C₁₋₇ alkyl group" represented by R⁹ include linear C₁₋₇ alkyl groups (e.g., methyl, ethyl, propyl, butyl, pentyl, hexyl, heptyl, etc.) and branched C₃₋₇ alkyl groups (e.g., isopropyl, isobutyl, sec-butyl, tert-butyl, isopentyl, neopentyl, etc.). Among these, branched C₃₋₇ alkyl groups are preferred. In particular, isopropyl is preferred.

Examples of the "substituents" in the "optionally substituted C₁₋₇ alkyl group" represented by R⁹ include (i) hydroxy group, (ii) C₁₋₇ acyloxy (e.g., C₁₋₆ alkyl-carbonyloxy such as acetoxy and propionyloxy; benzoyloxy, etc.), (iii) amino which may have one or two substituent(s) selected from the group consisting of C₁₋₆ alkoxy-carbonyl (e.g., methoxycarbonyl, ethoxycarbonyl, tert-butoxycarbonyl, etc.), benzyloxycarbonyl, C₁₋₃ acyl (e.g., C₁₋₂ alkyl-carbonyl such as acetyl and propionyl, etc.), C₁₋₃ alkylsulfonyl (e.g., methanesulfonyl, etc.), C₁₋₃ alkyl (e.g., methyl, ethyl, etc.) and the like (for example, amino, methoxycarbonylamino, ethoxycarbonylamino, tert-butoxycarbonylbenzyloxycarbonylamino, acetylamino, methanesulfonylamino, methylamino, dimethylamino and the like), (iv) C₁₋₁₀ (preferably C₁₋₄) alkoxy which may have 1 to 3 substituent(s) selected from the group consisting of C₃₋₇ cycloalkyloxycarbonyloxy (e.g., cyclohexyloxycarbonyloxy, etc.) and C₁₋₃ alkoxy (e.g., methoxy, ethoxy, etc.) (for example, methoxy, ethoxy, propoxy, tert-butoxy, cyclohexyloxycarbonyloxy-1-ethoxy, methoxymethoxy, ethoxymethoxy, etc.), (v) C₁₋₆ alkoxy-carbonyl (e.g., methoxycarbonyl, ethoxycarbonyl, propoxycarbonyl, etc.) and the like. Among these, hydroxy group is preferred.

Such "C₁₋₇ alkyl group" may have, for example, 1 to 5, preferably 1 to 3, substituent(s) mentioned above at positions where substitution is possible, and if the number of substituents is two or more, the respective substituents may be the same or different.

Examples of the "C₃₋₇ cycloalkyl group" in the "optionally substituted C₃₋₇ cycloalkyl group" represented by R⁹ include cyclopropyl, cyclobutyl, cyclopentyl, cyclohexyl, cycloheptyl and the like. Among these, cyclopropyl is preferred.

As the "substituent(s)" in the "optionally substituted C₃₋₇ cycloalkyl group" represented by R⁹, 1 to 3 substituent(s) as same as those in the "substituent(s)" in the "optionally substituted C₁₋₇ alkyl group" represented by R⁹ can be recited. When the number of substituents is two or more, the respective substituents may be the same or different.

Examples of the "C₁₋₆ alkoxyamino group" in the "optionally substituted C₁₋₆ alkoxyamino group" represented by R⁹ include mono- or di-C₁₋₆ alkoxyamino groups (e.g., methoxyamino, ethoxyamino, dimethoxyamino, diethoxyamino, ethoxymethoxyamino, etc.). Among these, mono-C₁₋₃ alkoxyamino groups (e.g., methoxyamino, etc.) are preferred.

As the "substituent(s)" in the "optionally substituted C₁₋₆ alkoxyamino group" represented by R⁹, the same number and the same kinds of substituent(s) as those in the "substituent(s)" in the "optionally substituted C₁₋₇ alkyl group" represented by R⁹ can be recited. When the number of substituents is two or more, the respective substituents may be the same or different. Such "substituent(s)" may substitute for "C₁₋₆ alkoxy group" or "nitrogen atom in an amino group" of C₁₋₆ alkoxyamino group.

Concrete examples of such "optionally substituted C₁₋₆ alkoxyamino group" include methoxyamino, N-methyl-N-methoxyamino, N-ethyl-N-methoxyamino, ethoxyamino, dimethoxyamino, diethoxyamino, ethoxymethoxyamino, and the like. Preferred examples include C₁₋₃ alkoxyamino groups, N-C₁₋₃ alkyl-N-C₁₋₃ alkoxyamino groups, and the like.

The "substituent(s)" in the "optionally substituted hydroxyamino group" represented by R⁹ may substitute for "hydroxy group" or "amino group" in a hydroxyamino group, and examples of the substituent on the "hydroxy group" include (i) C₁₋₇ acyl groups (e.g., C₁₋₆ alkyl-carbonyl such as acetyl and propionyl; benzoyl, etc.), (ii) amino groups which may have one or two substituent(s) selected from the group consisting of C₁₋₆ alkoxy-carbonyl (e.g., methoxycarbonyl, ethoxycarbonyl, tert-butoxycarbonyl, etc.), benzyloxycarbonyl, C₁₋₃ acyl (e.g., C₁₋₂ alkyl-carbonyl such as acetyl and propionyl), C₁₋₃ alkylsulfonyl (e.g., methane sulfonyl, etc.) and C₁₋₃ alkyl (e.g., methyl, ethyl, etc.) (for example, amino, methoxycarbonylamino, ethoxycarbonylamino, tert-butoxycarbonylbenzyloxycarbonylamino, acetylamino, methanesulfonylamino, methylamino, dimethylamino, etc.), (iii) C₁₋₁₀ (preferably C₁₋₄) alkyl groups which may have one to three substituent(s) selected from the group consisting of C₃₋₇ cycloalkyloxycarbonyloxy (e.g., cyclohexyloxycarbonyloxy, etc.) and C₁₋₃ alkoxy (e.g., methoxy, ethoxy, etc.) (for example, methyl, ethyl, propyl, tert-butyl, cyclohexyloxycarbonyloxy-1-ethyl, methoxymethyl, ethoxymethyl, etc.), and examples of the substituent(s) on the "amino group" include groups described in the above (i) to (iii). The respective substituents in the "hydroxy group" and "amino group" on the hydroxyamino group may be the same or different.

Preferred examples of the "optionally substituted hydroxyamino group" include N-C₁₋₆ alkyl-N-hydroxyamino groups (e.g., N-methyl-N-hydroxyamino, N-ethyl-N-hydroxyamino and the like). More preferred examples include N-C₁₋₃ alkyl-N-hydroxyamino groups.

Examples of the "C₁₋₇ alkyl group" in the "optionally substituted C₁₋₇ alkyl group" represented by R¹⁰ include linear or branched C₁₋₇ alkyl groups (e.g., methyl, ethyl, propyl, isopropyl, butyl, isobutyl, sec-butyl, tert-butyl, pentyl, isopentyl, neopentyl, hexyl, heptyl, etc.). Among these, C₁₋₃ alkyl groups (e.g., methyl, ethyl, propyl, isopropyl, etc.) are preferred. Isopropyl is particularly preferred.

As the "substituent(s)" in the "optionally substituted C₁₋₇ alkyl group" represented by R¹⁰, the same number and the same kinds of substituent(s) as those in the "substituent(s)" in the "optionally substituted C₁₋₇ alkyl group " represented by R⁹ can be recited. When the number of substituents is two or more, the respective substituents may be the same or different.

Examples of the "substituent(s)" in the "optionally substituted phenyl group" represented by R¹⁰ include halogens (e.g. fluorine, chlorine, bromine, iodine, etc.), C₁₋₃ alkyl groups (e.g., methyl, ethyl, propyl, isopropyl, etc.), C₁₋₃ alkoxy groups (e.g., methoxy, ethoxy, propoxy, isopropoxy, etc.). Among these halogens (preferably fluorine) are preferred.

Such "phenyl group" may have, for example, 1 to 5, preferably 1 to 3, substituent(s) mentioned above at positions where substitution is possible, and if the number of substituents is two or more, the respective substituents may be the same or different.

R⁹ is preferably a substituted branched C₃₋₇ alkyl group or a substituted C₃₋₇ cycloalkyl group, more preferably a branched C₃₋₇ alkyl group substituted by a hydroxy group or a C₃₋₇ cycloalkyl group substituted by a hydroxy group. Among these, C₃₋₇ cycloalkyl groups substituted by a hydroxy group are preferred. Also C₁₋₃ alkyl groups which may be substituted by a hydroxy group, C₃₋₇ cycloalkyl groups which may be substituted by a hydroxy group, as well as mono-C₁₋₃ alkoxyamino groups, N-C₁₋₃ alkyl-N-hydroxyamino groups, hydroxyamino group and the like are preferred. Especially preferred R⁹ is a cyclopropyl group, which may be substituted by a hydroxy group, or a methoxyamino group. A cyclopropyl group substituted by a hydroxy group is most preferred.

Preferably, R¹⁰ is an optionally substituted C₁₋₇ alkyl group. More preferably, R¹⁰ is a C₁₋₃ alkyl group which may be substituted by a hydroxy group or the like. Especially preferred R¹⁰ is isopropyl. Also phenyl is preferred.

Preferred examples of compound (VIII) are compounds wherein R⁹ is a C₁₋₃ alkyl group which may be substituted by a hydroxy group, a C₃₋₇ cycloalkyl group which may be substituted by a hydroxy group or a mono-C₁₋₃ alkoxyamino group; and R¹⁰ is a C₁₋₃ alkyl group or a phenyl group, and salts thereof.

As more preferably compounds, compounds wherein R⁹ is (1) a C₁₋₃ alkyl group substituted by one or two hydroxy group(s), (2) a C₃₋₇ cycloalkyl group substituted by a hydroxy group, or (3) a C₁₋₃ alkoxyamino group; and R¹⁰ is an isopropyl group or phenyl group, and salts thereof.

Concrete examples of compound (VIII) include 3-(N-benzyl-N-methylaminomethyl)-4,7-dihydro-5-isobutyryl-7-(2,6-difluorobenzyl)-2-(4-cyclopropanecarbonylaminophenyl)-4-oxothieno[2,3-b]pyridine,
5-benzoyl-3-(N-benzyl-N-methylaminomethyl)-7-(2,6-difluorobenzyl)-4,7-dihydro-4-oxo-2-[4-(3-hydroxy-2-methylpropionylamino)phenyl]thieno[2,3-b]pyridine,
5-(4-fluorobenzoyl)-3-(N-benzyl-N-methylaminomethyl)-7-(2,6-difluorobenzyl)-4,7-dihydro-4-oxo-2-(4-cyclopropane carbonylaminophenyl)thieno[2,3-b]pyridine,
3-(N-benzyl-N-methylaminomethyl)-4,7-dihydro-5-isobutyryl-7-(2,6-difluorobenzyl)-2-[4-(3-hydroxy-2-methylpropionylamino]phenyl]-4-oxothieno[2,3-b]pyridine,
3-(N-benzyl-N-methylaminomethyl)-4,7-dihydro-5-isobutyryl-7-(2,6-difluorobenzyl)-2-(4-N'-methoxyureidophenyl)-4-oxothieno[2,3-b]pyridine,
3-(N-benzyl-N-methylaminomethyl)-4,7-dihydro-5-isobutyryl-7-(2,6-difluorobenzyl)-2-[4-[(1-hydroxycyclopropyl)carbonylamino]phenyl]-4-oxothieno[2,3-b]pyridine,
(R)-4,7-dihydro-2-[4-(3-hydroxy-2-methylpropionylamino)phenyl]-7-(2,6-difluorobenzyl)-3-(N-benzyl-N-methylaminomethyl)-5-isobutyryl-4-oxothieno[2,3-b]pyridine,
4,7-dihydro-2-[4-(2-hydroxy-2-methylpropionylamino)phenyl]-7-(2,6-difluorobenzyl)-3-(N-benzyl-N-methylaminomethyl)-5-isobutyryl-4-oxothieno[2,3-b]pyridine,
4,7-dihydro-2-[4-(3-hydroxy-3-methylbutyrylamino)phenyl]-7-(2,6-difluorobenzyl)-3-(N-benzyl-N-methylaminomethyl)-5-isobutyryl-4-oxothieno[2,3-b]pyridine,
(R)-4,7-dihydro-2-[4-(2,3-dihydroxypropionylamino)phenyl]-7-(2,6-difluorobenzyl)-3-(N-benzyl-N-methylaminomethyl)-5-isobutyryl-4-oxothieno[2,3-b]pyridine,
3-(N-benzyl-N-methylaminomethyl)-5-benzoyl-7-(2,6-difluorobenzyl)-4,7-dihydro-2-[4-[(1-hydroxycyclopropyl)carbonylamino]phenyl]-4-oxothieno[2,3-b]pyridine or salts thereof.

Among them, 3-(N-benzyl-N-methylaminomethyl)-4,7-dihydro-5-isobutyryl-7-(2,6-difluorobenzyl)-2-[4-[(1-hydroxycyclopropyl)carbonylamino]phenyl]-4-oxothieno[2,3-b]pyridine or a salt thereof is preferred.

As salts of compound (I) and compound (VIII), physiologically acceptable acid addition salts are preferred. Examples of such salts include salts with inorganic acids (e.g., hydrochloric acid, hydrobromic acid, nitric acid, sulfuric acid, phosphoric acid, etc.), and salts with organic acids (e.g., formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.). When compound (I) has an acidic group, physiologically acceptable salts may be formed together with inorganic bases (e.g., alkaline metal salts or alkaline earth metals such as sodium, potassium, calcium, magnesium and the like, and ammonia and the like), or organic bases (e.g., trimethylamine, triethylamine, pyridine, picoline, ethanolamine, diethanolamine, triethanolamine, dicyclohexylamine, N,N'-dibenzylethylenediamine, etc.).

Compound (I) can be produced in accordance with a per se known method as disclosed, for example, in JP 9-169768 or WO 96/24597 or analogous methods thereto. As concrete examples, Production method 1 and Production method 2 described below can be recited. Compounds in any formulas may form salts, and examples of such salts include those as same as salts of compound (I).

### (Production Method 1)

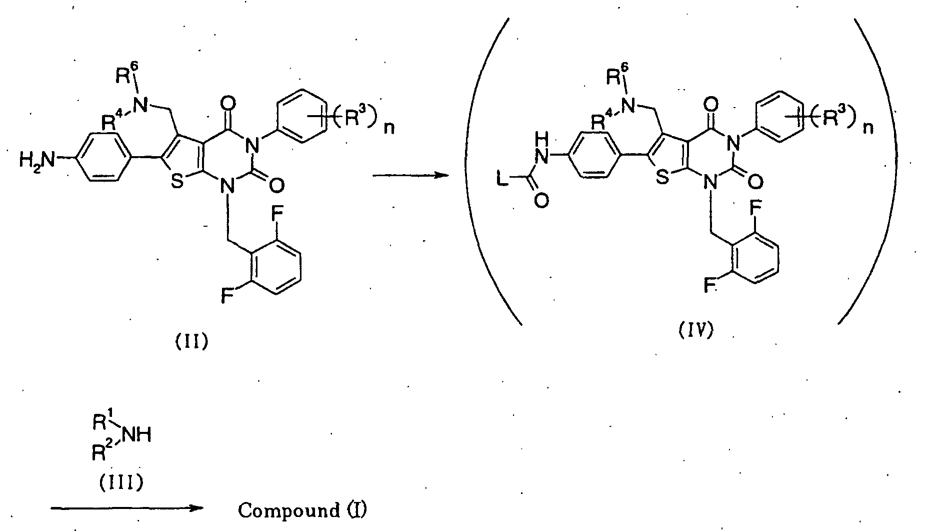

In the above formulae, L represents a leaving group, and other symbols are as defined above.

The "leaving group" for L includes, for example, 1-imidazolyl, a halogen atom, an alkoxy group which may have a substituent etc. The "alkoxy group which may have a substituent" includes, for example, C₁₋₄ alkoxy groups which may have 1 to 3 halogen atom(s) such as chlorine, bromine, etc. (e.g., 2,2,2-trichloroethoxy group, etc.).

Compound (II) can be produced by the methods as disclosed in JP 9-169768 or analogous methods thereto.

Compound (I) can be produced by reacting compound (II) with carbonyldiimidazole (N,N'-carbonyldiimidazole; CDI) or phosgene (including dimer and trimer) etc. to obtain compound (IV), followed by reacting with compound (III). The reaction can be carried out without isolation of compound (IV), or compound (IV) can be used as an isolated form in the next reaction.

Compound (IV) can be also produced by reacting compound (II) with, for example, a chloroformic acid ester compound (e.g., 2,2,2-trichloroethyl chloroformate, 1-chloroethyl chloroformate, etc.).

In the reaction of compound (II) with carbonyldiimidazole or phosgene, etc., carbonyldiimidazole or phosgene, etc. is used in amount of about 1 to 3 moles, relative to one mole of compound (II).

This reaction is advantageously carried out in a solvent which will not adversely affect the reaction.

Examples of such solvent include ethers (e.g., ethyl ether, dioxane, dimethoxyethane, tetrahydrofuran, etc.), aromatic hydrocarbons (e.g., benzene, toluene, etc.), amides (e.g., dimethylformamide, dimethylacetamide, etc.), halogenated hydrocarbons (e.g., chloroform, dichloromethane, etc.), and so on.

The reaction temperature is usually about 0 to about 150°C, preferably room temperature (about 15 to about 25°C). The reaction time is usually about 1 to about 36 hours.

This reaction is carried out in the presence of a base if necessary.

The "base" is exemplified by inorganic bases such as sodium carbonate, sodium hydrogencarbonate, potassium carbonate, potassium hydrogencarbonate, sodium hydroxide, potassium hydroxide and thallium hydroxide, and organic bases such as triethylamine and pyridine, etc.

The amount of the "base" is about 2 to 20 moles, preferably about 5 to 12 moles, relative to one mole of compound (II).

The subsequent reaction with compound (III) can be carried out under the same conditions as the above reaction of compound (II) with carbonyldiimidazole or phosgene. The amount of compound (III) is about 2 to 20 moles, preferably about 5 to 10 moles, relative to one mole of compound (II) or compound (IV). The reaction temperature is usually about 0 to 150°C, preferably room temperature (about 15 to 25°C). The reaction time is usually about 1 to 6 hours.

Compound (III) and carbonyldiimidazole or phosgene can be reacted with compound (II) at the same time.

### (Production Method 2)

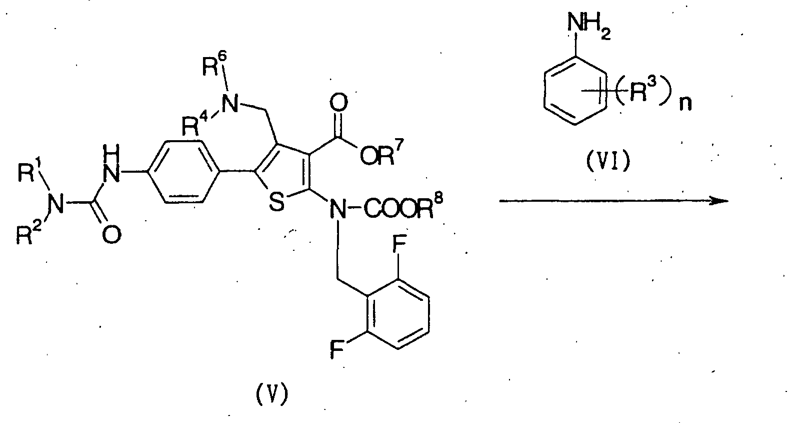

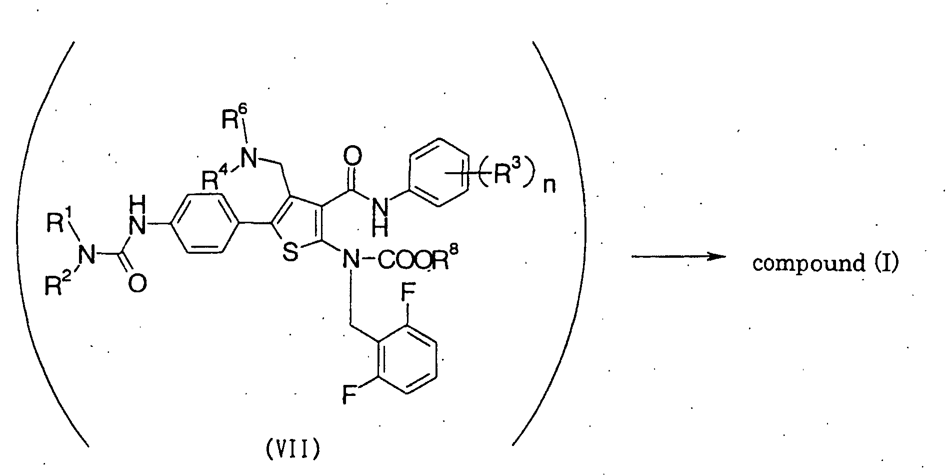

In the above formulae, R⁷ represents a hydrogen atom or an alkyl group, R⁸ represents an alkyl group, and other symbols are as defined above.

Examples of the "alkyl group" represented by R⁷ or R⁸ include those recited for the "C₁₋₄ alkyl group" of the "C₁₋₄ alkyl group which may have a substituent" represented by R¹ or R².

Compound (V) can be produced in any per se known manner, for example, p-hitrophenylacetone is reacted with a cyanoacetic acid ester derivative and sulfur [e.g., Chem. Ber., 99, 94-100(1966)], and thus obtained 2-amino-4-methyl-5-(4-nitrophenyl)thiophene is subjected to the methods disclosed in JP 9-169768, WO 96/24597 or analogous methods thereto.

1) When R⁷ is a hydrogen atom, compound (I) can be produced by reacting compound (V) with a compound of the formula: 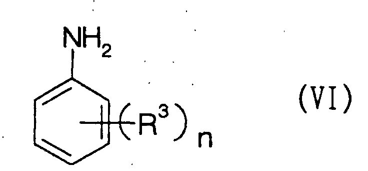 [wherein each symbol is as defined above], or a salt thereof [hereinafter, also abbreviated as compound (VI)], in the presence of a condensing agent, to obtain compound (VII), following by subjecting to cyclization.

The "condensing agent" includes, for example, benzotriazol-1-yloxytripyrrolidinophosphonium hexafluorophosphate (PyBOP), etc.

The amount of the "condensing agent" is about 1 to 3 moles, relative to one mole of compound (V).

This reaction is advantageously carried out in a solvent which will not adversely affect the reaction.

Examples of such solvent include alcohols (e.g., ethanol, methanol, etc.), aromatic hydrocarbons (e.g., benzene, toluene, etc.), amides (e.g., dimethylformamide, dimethylacetamide, etc.), halogenated hydrocarbons (e.g., chloroform, dichloromethane, etc.), and so on.

The reaction temperature is usually about 0 to about 150°C, preferably room temperature (about 15 to about 25°C). The reaction time is usually about 1 to about 36 hours.

The product as produced in the manner mentioned above may be applied to the next reaction in a reaction mixture or as a crude product, or may be isolated from the reaction mixture in any conventional manner.

Compound (VII) is subjected to cyclization in the presence of a base.

The "base" is exemplified by inorganic bases such as sodium methoxide, sodium carbonate, sodium hydrogencarbonate, potassium carbonate, potassium hydrogencarbonate, sodium hydroxide, potassium hydroxide and thallium hydroxide, and organic bases such as triethylamine and pyridine, etc.

The amount of the "base" is about 2 to 20 moles, preferably about 5 to 12 moles, relative to one mole of compound (VII).

This reaction is advantageously carried out in a solvent which dose not adversely affect the reaction.

Examples of such solvent include alcohols (e.g., ethanol, methanol, etc.), aromatic hydrocarbons (e.g., benzene, toluene, etc.), amides (e.g., dimethylformamide, dimethylacetamide, etc.), halogenated hydrocarbons (e.g., chloroform, dichloromethane, etc.), and so on.

The reaction temperature is usually about 0 to about 150°C, preferably room temperature (about 15 to about 25°C). The reaction time is usually about 1 to about 36 hours.

2) When R⁷ is an alkyl group, compound (I) can be produced by reacting compound (V) with an activated compound (VI).

Activated compound (VI) can be produced in any per se known manner, for example, by reacting an organoaluminum reagent with compound (VI) in a suitable solvent that does not adversely affect the reaction.

The "organoaluminum reagent" includes, for example, trimethyl aluminum, dimethyl aluminum chloride, etc, and a solution including them, etc.

The amount of the "organoaluminum reagent" is 1 to 5 moles, preferably about one mole, relative to one mole of compound (VI).

Preferable examples of the solvent include halogenated hydrocarbons (e.g., chloroform, dichloromethane, etc.), and so on.

The reaction temperature is usually about 0 to 150°C, preferably room temperature (about 15 to 25°C). The reaction time is usually about 1 to 6 hours.

The cyclization can be carried out by reacting compound (V) with an activated compound (VI) to obtain compound (I).

The amount of "compound (V)" is preferably about one fifth of the amount of mixture of compound (VI) and the organoaluminum reagent.

This reaction is advantageously carried out in a solvent which will not adversely affect the reaction.

Such a solvent is preferably the same as those used in the reaction to obtain an activated compound (VI).

The reaction temperature is usually about 0 to 150°C, preferably room temperature (about 15 to 25°C). The reaction time is usually about 1 to 48 hours.

Compound (I) may be isolated and purified by ordinary means of separation such as recrystallization, distillation and chromatography, etc.

When compound (I) is obtained in a free form, it can be converted to a salt by a method known per se or a method analogous thereto. When compound (I) is obtained in salt form, it can be converted to the free form or another salt by a method known per se or a method analogous thereto. Compound (I) may be a hydrate or a non-hydrate. The hydrate is exemplified by monohydrate, sesquihydrate and dihydrate. When compound (I) is obtained as a mixture of optically active substances, it can be resolved into the (R)- and (S)-forms by per se known optical resolution techniques. Compound (I) may be labeled with an isotope (e.g., ³H, ¹⁴C, ³⁵S), and the like.

Compound (VIII) or a salt thereof can be produced in any per se known manner, disclosed, for example in WO 95/28405, WO 00/00493 or analogous methods thereto.

As mentioned above, in the first embodiment of the granule of the present invention, a physiologically active substance and a cellulose-type disintegrant are essential, in the second embodiment, a physiologically active substance, a cellulose-type disintegrant and a binder are essential, and in the third embodiment, a cellulose-type disintegrant and a binder are not essential with regard to compound (I).

The aspects common to the production of the granules of these first to third embodiments are explained below.

A physiologically active substance, a mixture of a cellulose-type disintegrant (e.g., compound selected from CMC-Ca, carboxylmethylcellulose, croscarmellose sodium and low substituted hydroxypropyl cellulose or disintegrant comprising two or more compounds in combination and the like, preferably CMC-Ca and the like) and the like, and a binder (e.g., HPC, pre-gelatinized starch, sucrose, gelatin, powdered acacia, methylcellulose, hydroxypropyl methylcellulose, carboxymethylcellulose, sodium carboxymethylcellulose, polyvinylpyrrolidone, crystalline cellulose, dextrin, pullulan and the like, preferably HPC and the like) and the like are mixed, granulated to give granules, which are dried as necessary. The mixing and granulation can be done using a granulator generally employed.

For example, a physiologically active substance, an excipient (preferably lactose, starch and the like) and a cellulose-type disintegrant (preferably CMC-Ca) are mixed in advance and the obtained mixture (pre-mix) and a solution comprising a binder (preferably a solution of HPC and the like) are mixed and granulated. As the solution comprising a binder to be used here, an aqueous solution and an alcohol solution can be mentioned. Of these, an aqueous solution and an alcohol solution having 1 to 3 carbon atoms, particularly an aqueous solution and an ethanol solution, are preferable. The alcohol solution means a (water-alcohol) solution, wherein the proportion of alcohol in the solution is generally 5-99.5%, preferably 10-50%.

As the binder, any of a compound selected from HPC, pre-gelatinized starch, sucrose, gelatin, powdered acacia, methylcellulose, hydroxypropyl methylcellulose, carboxymethylcellulose, sodium carboxymethylcellulose, polyvinylpyrrolidone, crystalline cellulose, dextrin, pullulan and the like and a binder comprising two or more compounds in combination can be used.

In addition, the granule of the present invention can be obtained by the use of a suspension comprising a binder, instead of a solution comprising a binder. Alternatively, the granule can be produced by dissolving or suspending an excipient (preferably lactose, starch and the like), a disintegrant (preferably CMC-Ca and the like), a lubricant (preferably stearic acid, calcium stearate and the like), a coloring agent (preferably yellow ferric oxide, red ferric oxide and the like), flavor (preferably lemon flavor, lime flavor and the like), light-blocking agent (preferably titanium oxide, talc and the like), stabilizer (preferably ascorbic acid, sodium pyrosulfite, inorganic salts (magnesium carbonate, calcium carbonate and the like) and the like), a functional polymer (preferably hydroxymethylcellulose, ethylcellulose, acrylic resin and the like) and the like in a solution or suspension of a binder and mixing with a pre-mix.

The granule of the first embodiment of the present invention essentially comprises a physiologically active substance and a cellulose-type disintegrant. When the physiologically active substance is compound (I), i.e., the third embodiment, a granule can be obtained even when a cellulose-type disintegrant is not included.

The mixing and granulation of the physiologically active substance and a binder such as HPC are preferably conducted at about 20-45°C.

The content of the physiologically active substance in the granule of the present invention is not particularly limited as long as the object of the present invention can be achieved. It is, for example, 0.1-30 w/wt%, preferably 0.2-20 w/w%.

The amount of the "binder" to be used is not particularly limited as long as the object of the present invention can be achieved. It is, for example, about 0.01-100 parts by weight, preferably about 0.02-50 parts by weight, per 1 part by weight of the physiologically active substance, and the like.

The amount of the "excipient" to be used is not particularly limited as long as the object of the present invention can be achieved. It is, for example, about 0.25-1000 parts by weight, preferably about 0.3-600 parts by weight, more preferably about 0.5-150 parts by weight, per 1 part by weight of the physiologically active substance, and the like.

The amount of the "disintegrant" of the cellulose-type disintegrant and the like to be used is not particularly limited as long as the object of the present invention can be achieved. It is, for example,.about 0.001-300 parts by weight, preferably about 0.05-250 parts by weight, per 1 part by weight of the physiologically active substance, and the like. When the physiologically active substance is compound (I), the granule can be produced even without the use of a disintegrant, not to mention a cellulose-type disintegrant.

The amount of the cellulose-type disintegrant and the binder to be used in the second embodiment are the same as those of the aforementioned disintegrant and binder.

The content ratio of the "excipient" optionally dissolved or suspended further in a solution or suspension comprising a binder is not particularly limited as long as the object of the present invention can be achieved. It is, for example, about 1-50% and the like.

The content ratio of the "disintegrant" optionally dissolved or suspended further in a solution or suspension comprising a binder is not particularly limited as long as the object of the present invention can be achieved. It is, for example, about 1-50% and the like.

The content ratio of the "lubricant" optionally dissolved or suspended further in a solution or suspension comprising a binder is not particularly limited as long as the object of the present invention can be achieved. It is, for example, about 0.05-20% and the like.

The content ratio of the "coloring agent" optionally dissolved or suspended further in a solution or suspension comprising a binder is not particularly limited as long as the object of the present invention can be achieved. It is, for example, about 0.01-10% and the like.

The content ratio of the "flavor" optionally dissolved or suspended further in a solution or suspension comprising a binder is not particularly limited as long as the object of the present invention can be achieved. It is, for example, about 0.01-10% and the like.

The content ratio of the "light-blocking agent" optionally dissolved or suspended further in a solution or suspension comprising a binder is not particularly limited as long as the object of the present invention can be achieved. It is, for example, about 0.05-20% and the like.

The content ratio of the "stabilizer" optionally dissolved or suspended further in a solution or suspension comprising a binder is not particularly limited as long as the object of the present invention can be achieved. It is, for example, about 0.01-30% and the like.

The content ratio of the "functional polymer" optionally dissolved or suspended further in a solution or suspension comprising a binder is not particularly limited as long as the object of the present invention can be achieved. It is, for example, about 1-90% and the like.

The particle size (diameter) of the obtained granule is preferably about 50-1000 µm and the like.

When a solution comprising a binder is used, the obtained granules are dried generally at about 30-60°C for about 0.5-30 minutes to remove the solvent and to remove water in the granules.

The granule of the present invention is superior for tableting and preferably used for tableting.

Granules can be adjusted by mixing and drying with a solution or suspension comprising a binder and other components by a conventional wet granulation method and preferably obtained by fluidized bed granulation. A mixture (pre-mix) obtained by mixing in advance a physiologically active substance, an excipient and a cellulose-type disintegrant as necessary, such as CMC-Ca and the like, and mixed and dried while spraying as necessary a solution or suspension comprising a binder for granulation. The conditions are as follows.
air supply quantity (aeration): 1-10 m³/min
spraying temperature: 25-100°C
spraying rate: 10-800 g/min
drying rate: 30-90°C
drying period: 0.5-30 min

The solid preparation of the present invention comprises the aforementioned granule. Preferably, it comprises the aforementioned granule, a cellulose-type disintegrant (e.g., CMC-Ca, croscarmellose sodium, or low substituted hydroxypropyl and the like, preferably CMC-Ca and the like) CMC-Ca and a stearic acid-type lubricant (e.g., stearic acid, sodium stearate, magnesium stearate and the like, preferably magnesium stearate and the like).

The content of the physiologically active substance in the solid preparation of the present invention is not particularly limited as long as the object of the present invention is achieved. It is, for example, about 0.1-40 w/w%, preferably about 0.3-35 w/w%, more preferably about 1-30 w/w% and the like.

The content of the cellulose-type disintegrant (total amount including that in the granule) in the solid preparation of the present invention is not particularly limited as long as the object of the present invention is achieved. It is, for example, about 0.1-15 w/w%, preferably about 1-12 w/w%, more preferably about 3-10 w/w% and the like.

The content of the stearic acid-type lubricant in the solid preparation of the present invention is not particularly limited as long as the object of the present invention is achieved. It is, for example, about 0.3-1.5 w/w%, preferably about 0.5-1.2 w/w% and the like.

The solid preparation of the present invention may comprise conventional excipient, disintegrant, binder, lubricant, coloring agent, flavor, light-blocking agent and the like as materials of pharmaceutical preparation.

Examples of the "excipient" include lactose, starch, sucrose, mannitol, crystalline cellulose, light anhydrous silicic acid, magnesium carbonate, calcium carbonate, calcium phosphate, calcium sulfate, aluminum silicate, aluminum metasilicate and the like.

Examples of the "disintegrant" include CMC-Ca, croscarmellose sodium, sodium carboxymethyl starch, starch, low substituted hydroxypropyl cellulose, cross-linked insoluble polyvinylpyrrolidone and the like.

Examples of the "binder" include HPC, pre-gelatinized starch, sucrose, gelatin, powdered acacia, methylcellulose, hydroxypropyl methylcellulose, carboxymethylcellulose, sodium carboxymethylcellulose, polyvinylpyrrolidone, crystalline cellulose, dextrin, pullulan and the like. Of these, preferred is HPC.

When HPC is used as a binder, an HPC solution (e.g., aqueous solution, alcohol solution and the like) may be used. Of these, an alcohol solution of HPC is preferable. Concrete examples thereof include an about 1-100-fold diluted HPC or aqueous HPC solution with alcohols (e.g., ethanol, isopropyl alcohol, n-propyl alcohol, methanol and the like), and the like. When HPC is to be used, an HPC suspension may be used.

Examples of the "lubricant" include stearic acid, calcium stearate, magnesium stearate, talc, colloidal silica and the like.

Examples of the "coloring agent" include yellow ferric oxide, red ferric oxide and the like. Of these, preferred is yellow ferric oxide.

As the "flavor", both a synthetic substance and natural products may be used. Examples thereof include lemon flavor, lime flavor, orange flavor, strawberry flavor, menthol and the like.

Examples of the "light-blocking agent" include titanium oxide, talc, calcium carbonate, magnesium carbonate and the like. Of these, preferred is titanium oxide.

The content of the excipient in the solid preparation of the present invention is not particularly limited as long as the object of the present invention is achieved. It is, for example, about 20-99.9 w/w%, preferably about 40-95 w/w% and the like.

The content of the disintegrant in the solid preparation of the present invention is not particularly limited as long as the object of the present invention is achieved. It is, for example, about 0.1-30 w/w%, preferably about 3-25 w/w% and the like.

The content of the binder in the solid preparation of the present invention is not particularly limited as long as the object of the present invention is achieved. It is, for example, 1-10 w/w%, preferably about 2-5 w/w% and the like.

The content of the lubricant in the solid preparation of the present invention is not particularly limited as long as the object of the present invention is achieved. It is, for example, about 0.1-3 w/w%, preferably about 0.2-2 w/w% and the like.

The content of the coloring agent in the solid preparation of the present invention is not particularly limited as long as the object of the present invention is achieved. It is, for example, about 0.0001-0.4 w/w%, preferably about 0.001-0.2 w/w% and the like.

The content of the flavor in the solid preparation of the present invention is not particularly limited as long as the object of the present invention is achieved. It is, for example, about 0.0001-0.4 w/w%, preferably about 0.001-0.2 w/w% and the like.

The content of the light-blocking agent in the solid preparation of the present invention is not particularly limited as long as the object of the present invention is achieved. It is, for example, about 0.001-4 w/w%, preferably about 0.003-3 w/w% and the like.

Preferable examples of the solid preparation of the present invention are:
(1) a solid preparation comprising a granule comprising compound (I),
(2) a solid preparation comprising a granule comprising compound (I) and HPC,
(3) a solid preparation comprising a granule comprising compound (I) and HPC, CMC-Ca and magnesium stearate,
(4) a solid preparation comprising a granule comprising compound (I) and HPC, CMC-Ca, magnesium stearate, lactose and starch,
(5) a solid preparation comprising a granule comprising compound (I) and HPC; CMC-Ca, magnesium stearate, lactose, starch and hydroxypropyl methylcellulose,
(6) a solid preparation comprising a granule comprising compound (I) and HPC, CMC-Ca, magnesium stearate, lactose, starch, hydroxypropyl methylcellulose and titanium oxide,
(7) a solid preparation comprising a granule comprising compound (I) and HPC, CMC-Ca, magnesium stearate, HPC, lactose, starch, hydroxypropyl methylcellulose, titanium oxide and yellow ferric oxide,
(8) a solid preparation comprising a granule comprising compound (I) or compound (VIII) and CMC-Ca,
(9) a solid preparation comprising a granule comprising compound (I) or compound (VIII), CMC-Ca and HPC, CMC-Ca and magnesium stearate,
(10) a solid preparation comprising a granule comprising compound (I) or compound (VIII), CMC-Ca and HPC, CMC-Ca, magnesium stearate, lactose and starch,
(11) a solid preparation comprising a granule comprising compound (I) or compound (VIII), CMC-Ca and HPC, CMC-Ca, magnesium stearate, lactose, starch and hydroxypropyl methylcellulose,
(12) a solid preparation comprising a granule comprising compound (I) or compound (VIII), CMC-Ca and HPC, CMC-Ca, magnesium stearate, lactose, starch, hydroxypropyl methylcellulose and titanium oxide,
(13) a solid preparation comprising a granule comprising compound (I) or compound (VIII), CMC-Ca and HPc, CMC-Ca, magnesium stearate, lactose, starch, hydroxypropyl methylcellulose, titanium oxide and yellow ferric oxide and the like.

The solid preparation of the present invention is produced by, for example, the methods shown below.
1) The aforementioned granule is sized to give a sized powder. For sizing, a commercially available sizer can be used generally, such as Power Mill and the like.
2) The sized powder obtained in the aforementioned 1), CMC-Ca and magnesium stearate are admixed with, if necessary, a disintegrant, a binder, a lubricant, a coloring agent, a flavor and a light-blocking agent to give a powder mixture.
3) The powder mixture obtained in the aforementioned 2) is formed to give a solid preparation.

The powder mixture obtained above may be used (i) as it is as a granule or fine granule, (ii) as a capsule upon filling in a capsule (e.g., gelatin capsule, hydroxypropyl methylcellulose capsule and the like), (iii) as a dry syrup to be suspended when in use, or (iv) as Caplet. Generally, the powder mixture is formed into a pill, a tablet and the like. Of these, tablet is preferable, round tablet, oval tablet and the like are more preferable, and oval tablet is particularly preferable.

The round tablet means a tablet having a round horizontal section in the width direction, wherein the corner of the tablet may be square or round.

The oval tablet means a tablet having an oval horizontal section in the width direction, wherein the corner of the tablet may be square or round.

The round tablet generally has a diameter of about 5-13 mm and a thickness of about 3-8 mm at the maximum sectional area in the width direction thereof. The oval tablet generally has a longer diameter of about 8-17 mm, a shorter diameter of about 5-9 mm and a thickness of about 3-8 mm at the maximum sectional area in the width direction thereof, wherein the corner of the tablet may be square or round.

For forming, a commercially available forming machine such as a tableting machine and the like can be used.

The punching pressure applied for forming tablets is generally 0.5-2.5 ton/cm².
4) The solid preparation obtained in the aforementioned 3) is processed as necessary to give a film-coated tablet.

To be specific, a film-coating liquid is spray on the solid preparation obtained in the aforementioned 3) (preferably tablet, more preferably oval tablet) and dried as necessary.

The "film-coating liquid" is prepared by, for example, dissolving or suspending a polymer for film-coating in a solvent. The film-coating liquid may further comprise, for example, a coloring agent (preferably yellow ferric oxide), a light-blocking agent (preferably titanium oxide) and the like.

Examples of the "polymer for film-coating" include hydroxypropyl methylcellulose, ethylcellulose, hydroxymethylcellulose, hydroxypropyl cellulose, hydroxypropyl methylcellulose acetate succinate, acrylic resin (methacrylic acid, acrylic copolymer, aminoalkylmethacrylate polymer etc.), shellac, polyvinylacetate phthalate, gum arabic, cellulose acetate phthalate, hydroxypropyl methylcellulose phthalate, carboxymethylethylcellulose and the like.

Examples of the "solvent" include water, alcohols (e.g., ethanol, isopropyl alcohol, n-propyl alcohol, methanol and the like), acetone, ethyl acetate, dichloromethane, chloroform, hexane, toluene, heptane and the like.

The amount of the "polymer for film-coating" to be used may be determined according to the kind of the solid preparation. When the solid preparation is a tablet, for example, it is about 0.5-10 w/w% of the tablet.

The spray temperature is generally 25-80°C.

The spray time is generally 5 minutes-24 hrs.

The drying temperature is generally 30-80°C.

The drying time is generally 1 minute-24 hrs.

The present invention also provides a film-coated tablet comprising compound A or compound B to be mentioned below.

The "film-coated tablet comprising compound A or compound B" can be produced by a method similar to the aforementioned method.

The solid preparation of the present invention can comprise a large content of a physiologically active substance, and is superior in disintegrability and dissolution of a physiologically active substance from the preparation. Particularly, a film-coated tablet can be preserved stably for a long period even not protected from light. In addition, by granulating a physiologically active substance and a liquid mixture of an alcohol solution and a binder, the granulation efficacy can be improved and granules having a pre-determined particle size can be prepared easily.

With low toxicity, the solid preparation of the present invention can be used in mammals (e.g., human, bovine, pig, dog, cat, mouse, rat, rabbit, etc.) as safe pharmaceutical agents and the like.

The solid preparation of the present invention may be used as preventive and therapeutic for various diseases and the like agents in accordance with the kind of the comprised physiologically active substance. For example, in the case where the physiologically active substance is a GnRH antagonist, the solid preparation can be used for the prophylaxis or treatment of male hormone or female hormone-dependent diseases, and for the prophylaxis or treatment of the diseases caused by excess secretion of these hormones, by suppressing the secretion of gonadotropin and by controlling the concentration of sex hormone in blood. To be specific, the solid preparation of the present invention is useful for preventing and/or treating sex hormone-dependent cancers (e.g., prostatic cancer, uterine cancer, breast cancer, pituitary tumor, etc.), bone metastasis of the sex hormone-dependent cancers, prostatic hypertrophy, hysteromyoma, endometriosis, precocious puberty, amenorrhea, premenstrual syndrome, multilocular ovary syndrome, pimples, alopecia, Alzheimer's disease (Alzheimer's disease, Alzheimer's senile dementia and mixed type thereof), etc. The solid preparation of the present invention is also useful for the regulation of reproduction in males and females (e.g., pregnancy regulators, menstrual cycle regulators, etc.). The solid preparation of the present invention may be also used as a male or female contraceptive, or as a female ovulation inducer. Based on its rebound effect after withdrawal, the solid preparation of the present invention can be used to treat infertility. Also the solid preparation of the present invention can be used for preventing and/or treating benign or malignant tumor which is independent of sex hormone and sensitive to LH-RH.

In addition, the solid preparation of the present invention is useful for regulation of animal estrus, improvement of meat quality and promotion of animal growth in the field of animal husbandry. The solid preparation of the present invention can be also useful as a fish-spawning promoter.

The solid preparation of the present invention can be also used to suppress the transient rise in plasma testosterone (in the case of male) concentration (flare phenomenon) observed in administration of a GnRH super-agonist such as leuprorelin acetate. The solid preparation of the present invention can be used in combination with a GnRH super-agonist such as leuprorelin acetate, gonadrelin, buserelin, triptorelin, goserelin, nafarelin, histrelin, deslorelin, meterelin, lecirelin, and so on. Among them, preferred is leuprorelin acetate.

It is also beneficial to use the solid preparation of the present invention in combination with at least one member selected from among the steroidal or nonsteroidal androgen antagonist or anti-estrogen agent, chemotherapeutic agent, GnRH antagonistic peptide, 5α-reductase inhibitor, α-receptor inhibitor, aromatase inhibitor, 17β-hydroxysteroid dehydrogenase inhibitor, adrenal androgen production inhibitor, phosphorylase inhibitor, drug for hormone therapy, and drug antagonizing growth factor or its receptor, among others.

Examples of the "chemotherapeutic agent" include ifosfamide, UFT, adriamycin, peplomycin, cisplatin, cyclophosphamide, 5-FU, UFT, methotrexate, mitomycin C, mitoxantrone, taxotere, and the like.

Examples of the "GnRH antagonistic peptide" include parenteral GnRH antagonistic peptides such as cetrorelix, ganirelix, abarelix, and the like.

Examples of the "adrenal androgen production inhibitor" include lyase (C_{17,20} -lyase) inhibitors, and the like.

Examples of the "phosphorylase inhibitor" include tyrosine phosphorylase inhibitor, and the like.

Examples of the "drugs for hormone therapy" include anti-estrogens, progesterons (e.g., MPA, etc.), androgens, estrogens and androgen antagonists, and the like.

The "growth factor" may be any substance that promotes proliferation of cells and generally includes peptides with molecular weights not more than 20,000 which express the action at low concentrations through binding to receptors. Specifically, there can be mentioned (1) EGF (epidermal growth factor) or substances having the substantially the same activity (e.g., EGF, heregulin (HER2 ligand), etc.), (2) insulin or substances having substantially the same activity (e.g., insulin, IGF (insulin-like growth factor)-1, IGF-2, etc.), (3) FGF (fibroblast growth factor) or substances having substantially the same activity (αFGF, βFGF, KGF (keratinocyte growth factor), HGF (hepatocyte growth factor), FGF-10, etc.), and (4) other growth factors (e.g., CSF (colony stimulating factor), EPO (erythropoietin), IL-2 (interleukin-2), NGF (nerve growth factor), PDGF (platelet-derived growth factor) and TGFβ (transforming growth factor β), etc.), among others.

The "growth factor receptor" may be any receptor capable of binding the growth factor, including EGF receptor, heregulin receptor (HER2), insulin receptor-1, insulin receptor-2, IGF receptor, FGF receptor-1, FGF receptor-2, etc.

Examples of the drug antagonizing the growth factor include herceptin (HER2 receptor antibody) and the like.

Examples of the drug antagonizing the growth factor or its receptor include herbimycin, PD153035 (see Science, 265 (5175) p.1093, (1994)) and the like.

As a further class of drugs antagonizing the growth factor or its receptor includes HER2 antagonists. The HER2 antagonist may be any substance that inhibits the activity of HER2 (e.g., phosphorylating activity), thus including an antibody, a low molecular weight compound (synthetic or natural product), an anti-sense, a HER2 ligand, heregulin, and any of them as partially modified or mutated in structure. Moreover, it may be a substance which inhibits HER2 activity by antagonizing HER2 receptor (e.g. HER2 receptor antibody). Examples of the low molecular weight compound having HER2 antagonizing activity include, for example, compounds described in WO 98/03505, namely 1-[3-[4-[2-((E)-2-phenylethenyl)-4-oxazolylmethoxy]phenyl]propyl]-1,2,4-triazole and so on.

For prostatic hypertrophy, examples of such combination include the solid preparation of the present invention in combination with the GnRH super-agonist, androgen antagonist, anti-estrogen agent, GnRH antagonistic peptide, 5α-reductase inhibitor, α-receptor inhibitor, aromatase inhibitor, 17β-hydroxysteroid dehydrogenase inhibitor, adrenal androgen production inhibitor, phosphorylase inhibitor, and so on.

For prostatic cancer, examples of such combination include the solid preparation of the present invention in combination with the GnRH super-agonist, androgen antagonist, anti-estrogen agent, chemotherapeutic agent (e.g., ifosfamide, UFT, adriamycin, peplomycin, cisplatin, etc.), GnRH antagonistic peptide, aromatase inhibitor, 17β-hydroxysteroid dehydrogenase inhibitor, adrenal androgen production inhibitor, phosphorylase inhibitor, drug for hormone therapy such as estrogens (e.g., DSB, EMP, etc.), androgen antagonist (e.g., CMA. etc.), drug antagonizing growth factor or its receptor, and so on.

For breast cancer, examples of such combination includes the solid preparation of the present invention in combination with the GnRH super-agonist, anti-estrogen, chemotherapeutic agent (e.g., cyclophosphamide, 5-FU, UFT, methotrexate, adriamycin, mitomycin C, mitoxantrone, etc.), GnRH antagonistic peptide, aromatase inhibitor, adrenal androgen production inhibitor, phosphorylase inhibitor, drug for hormone therapy such as anti-estrogens (e.g., tamoxifen, etc.), progesterones (e.g., MPA, etc.), androgens, estrogens, etc., drug antagonizing growth factor or its receptor, and so on.

The aforementioned drugs may be administered to the same subject concurrently with the solid preparation of the present invention, or after some interval. Alternatively, the aforementioned drugs (1 to 3 kinds of drugs) may be comprised in the solid preparation of the present invention. Furthermore, the solid preparation of the present invention may be administered prior to administration of the GnRH super-agonist such as leuprorelin acetate so as to conduct a treatment while preventing occurrence of flare phenomenon.

The solid preparation of the present invention is generally administered orally. While the dose of the solid preparation of the present invention varies depending on the kind and content of a physiologically active substance, dosage form, the time of sustained release of the physiologically active substance, target disease, target animal and the like, it may be an effective amount of the physiologically active substance. For example, when compound (I) or compound (VIII) is used as a physiologically active substance, the dose of the compound per administration is about 0.01-10 mg/kg body weight for an adult (body weight 60 kg).

The administration frequency is once in 2 days or 1 to 4 times a day, and the like.

### Examples

The present invention is explained in detail in the following by referring to Reference Examples, Examples and Experimental Examples, which are not to be construed as limitative.

### Reference Example 1

### 2-Amino-4-methyl-5-(4-nitrophenyl)thiophene-3-carboxylic acid ethyl ester

A mixture of 4-nitrophenylacetone (35.0 g, 195 mmol), ethyl cyanoacetate (23.8 g, 195 mmol), ammonium acetate (3.1 g, 40 mmol) and acetic acid (9.1 mL, 159 mmol) was refluxed for 24 hours while removing water using a Dean-Stark apparatus. After cooling, the reaction mixture was concentrated under reduced pressure and the residue was partitioned with dichloromethane and aqueous solution of sodium bicarbonate. After washing the organic layer with brine and drying over MgSO₄, the solvent was distilled off under reduced pressure. The residue was purified on a silica gel column chromatography. The resultant oily product was dissolved in ethanol and sulfur (5.0 g, 160 mmol) and diethyl amine (16.0 mL, 160 mmol) were added thereto, and the mixture was stirred at 60 to 70°C for 2 hours. After cooling, the reaction mixture was concentrated under reduced pressure, and the residue was partitioned with dichloromethane and aqueous solution of sodium bicarbonate. After washing the organic layer with brine and drying over MgSO₄, the solvent was distilled off under reduced pressure. The residue was purified on a silica gel column chromatography, and crystallized from ether-hexane, to give the title compound as red sheet crystals (22.2 g, 52%).
mp: 168-170°C (recrystallized from ether-hexane)

| Elemental Analysis for C₁₄H₁₄N₂O₄S; | | | |
|---|---|---|---|
| | C(%) | H(%) | N(%) |
| Calcd. | 54.89; | 4.61; | 9.14 |
| Found | 54.83; | 4.90; | 9.09 |

¹H-NMR(200 MHz, CDCl₃)δ: 1.39 (3H, t, J = 7.1 Hz), 2.40 (3H, s), 4.34 (2H, q, J = 7.1 Hz), 6.27(2H, br), 7.48 (2H, d, J = 8.7 Hz), 8.23 (2H, d, J = 8.7 Hz).
IR (KBr): 3446, 3324, 1667, 1580, 1545, 1506, 1491, 1475, 1410, 1332 cm⁻¹.

### Reference Example 2

### 5-Methyl-6-(4-nitrophenyl)-3-phenylthieno[2,3-d]pyrimidine-2,4(1H,3H)-dione

Phenylisocyanate (2.66 mL, 24.48 mmol) was added to a solution of the compound obtained in Reference example 1 (5.00 g, 16.32 mmol) in pyridine (30 mL). The mixture was stirred at 45°C for 6 hours and concentrated under reduced pressure to give a residue, which was then rendered a solution in ethanol (6 mL). To this solution, 28% sodium methoxide (7.86 g, 40.80 mmol) was added, and the mixture was stirred at the room temperature for 2 hours, followed by addition of 2N hydrochloric acid (25 mL, 50 mmol) and distillation of ethanol solvent under reduced pressure. The resultant residue was filtered, washed with water-ethanol and recrystallized from ethanol after drying under reduced pressure to give the title compound as yellow powder (6.09 g, 98%).
mp: >300°C

| Elemental Analysis for C₁₉H₁₃N₃O₄S.0.3H₂O | | | |
|---|---|---|---|
| | C(%) | H(%) | N(%) |
| Calcd. | 59.30; | 3.56; | 10.92 |
| Found | 59.56; | 3.52; | 10.93 |

¹H-NMR(300 MHz, DMSO-d₆) δ: 2.50 (3H, s), 7.31-7.46 (5H, m), 7.78(2H, d, J = 8.8 Hz), 8.32 (2H, d, J = 8.8 Hz), 12.50 (1H, s).
IR(KBr): 1715, 1657, 1593, 1510 cm⁻¹.

### Reference Example 3

### 1-(2,6-Difluorobenzyl)-5-methyl-6-(4-nitrophenyl)-3-phenylthieno[2,3-d]pyrimidine-2,4(1H,3H)-dione

Potassium carbonate (19.00 g, 0.138 mol), potassium iodide (22.90 g, 0.138 mol) and 2,6-difluorobenzyl chloride (22.40 g, 0.138 mol) were added to a solution of the compound obtained in Reference example 2 (52.54 g, 0.131 mol) in dimethylformamide (1.0 L), and the mixture was stirred at room temperature for 2 hours. A residue obtained after concentration of the reaction mixture was partitioned with chloroform and brine. The water layer was extracted with chloroform, and the combined extract was washed with brine and dried over MgSO₄, followed by distillation of solvent under reduced pressure. The resultant residue was purified on a silica gel column chromatography, to give the title compound as pale yellow crystals (61.50 g, 93%).
mp: 280-282°C

| Elemental Analysis: for C₂₆H₁₇N₃O₄SF₂ | | | |
|---|---|---|---|
| | C(%) | H(%) | N(%) |
| Calcd. | 61.78; | 3.39; | 8.31 |
| Found | 61.67; | 3.46; | 8.21 |

¹H-NMR(300 MHz, CDCl₃) δ: 2.57 (3H, s), 5.38 (2H, s), 6.94 (2H, d, J = 8.1 Hz), 7.42-7.58 (8H, m), 8.29 (2H, d, J = 8.8 Hz).
IR(KBr): 1719, 1669, 1524, 1473 cm⁻¹

### Reference Example 4

### 5-Bromomethyl-1-(2,6-difluorobenzyl)-6-(4-nitrophenyl)-3-phenylthieno[2,3-d]pyrimidine-2,4(1H,3H)-dione

A mixture of the compound obtained in Reference example 3 (30.34 g, 0.060 mol), N-bromosuccinimide (12.81 g, 0.072 mol), α,α'-azobisisobutyronitrile (1.15 g, 0.007 mol) and chlorobenzene (450 mL) was stirred at 85°C for 3 hours. After cooling, the reaction mixture was washed with brine and dried over MgSO₄, followed by distillation of the solvent under reduced pressure. The resultant residue was recrystallized from ethyl acetate to give the title compound as yellow needle crystals (80.21 g, 100%).
mp: 228-229°C
¹H-NMR(300 MHz, CDCl₃) δ: 4.77 (2H, s), 5.38 (2H, s), 6.96 (2H, t, J = 8.1 Hz), 7.29-7.58 (6H, m), 7.79 (2H, d, J = 8.5 Hz), 8.35 (2H, d, J = 8.5 Hz).
IR(KBr): 1721, 1680, 1524, 1473, 1348 cm⁻¹.
FAB-Mass m/z 584 (MH)⁺

### Reference Example 5

### 5-(N-Benzyl-N-methylaminomethyl)-1-(2,6-difluorobenzyl)-6-(4-nitrophenyl)-3-phenylthieno[2,3-d]pyrimidine-2,4(1H,3H)-dione

Ethyldiisopropylamine (27.00 mL, 0.155 mol) and benzylmethylamine (18.45 mL, 0.143 mol) were added to a solution of the compound obtained in Reference example 4 (80.00 g, 0.119 mol) in dimethylformamide (600 mL) under cooling with ice. After stirring at room temperature for 2 hours, the residue obtained by concentration of the reaction mixture was partitioned with ethyl acetate and saturated aqueous solution of sodium bicarbonate. The water layer was extracted with ethyl acetate, and the combined organic layer was dried over MgSO₄, followed by distillation of the solvent under reduced pressure. The resultant residue was purified on a silica gel column chromatography to give a yellow oily substance (74.90 g, 100%), which was recrystallized from ethyl acetate to give the title compound as yellow needle crystals.
mp: 173-174°C

| Elemental Analysis for C₃₄H₂₆N₄O₄SF₂.0.5H₂O | | | |
|---|---|---|---|
| | C(%) | H(%) | N(%) |
| Calcd. | 64.45; | 4.29; | 8.84 |
| Found | 64.50; | 4.24; | 8.82 |

¹H-NMR(300 MHz, CDCl₃) [free amine] δ: 1.31 (3H, s), 3.60 (2H, s), 3.96 (2H, s), 5.39 (2H, s), 6.95 (2H, t, J = 8.2 Hz), 7.18-7.55 (11H, m), 8.02 (2H, d, J = 9.0 Hz), 8.26 (2H, d, J = 9.0 Hz).
IR(KBr)[hydrochloride]: 1719, 1678, 1597, 1520 cm⁻¹.

### Reference Example 6

### 6-(4-Aminophenyl)-5-(N-benzyl-N-methylaminomethyl)-1-(2,6-difluorobenzyl)-3-phenylthieno[2,3-d]pyrimidine-2,4(1H,3H)-dione

1M Hydrogen chloride-ether (14.4 mL, 14.4 mmol) and 10% palladium carbon powder (300 mg) was added to a solution of the compound obtained in Reference example 5 (3.00 g, 4.80 mmol) in formic acid (30 mL) under ice-cooling, and the mixture was stirred at room temperature for 2 hours under normal pressures for allowing hydrogenation. The reaction mixture was filtered through Celite, and the residue obtained by concentration of filtrate under reduced pressure was partitioned with dichloromethane and saturated aqueous solution of sodium bicarbonate. The water layer was extracted with dichloromethane, and the combined organic layer was dried over MgSO₄, followed by distillation of the solvent under reduced pressure. The resultant residue was purified on a silica gel column chromatography to give the title compound as white crystals (2.41 g, 84%).
mp: 205-207°C

| Elemental Analysis: C₃₄H₂₈N₄O₂SF₂.0.1AcOEt.1. 2H₂O | | | |
|---|---|---|---|
| | C(%) | H(%) | N(%) |
| Calcd. | 66.09; | 5.03; | 8.96 |
| Found | 66.93; | 4.94; | 8.67 |

¹H-NMR(300 MHz, CDCl₃) δ: 2.05(3H, s), 3.56 (2H, s), 3.83 (2H, br), 3.88 (2H, s), 5.36 (2H, s), 6.70 (2H, d, J = 8.8 Hz), 6.88-6.94 (2H, m), 7.21-7.31 (8H, m), 7.41-7.53 (5H, m).
IR(KBr): 1715, 1657, 1628, 1537 cm⁻¹.

### Reference Example 7

### 5-(N-Benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione

Triethylamine (2.34 mL, 16.82 mmol) was added to a solution of the compound obtained in Reference example 6 (5.0 g, 8.41 mmol) in dichloromethane (120 mL) under ice-cooling and the mixture was stirred. To this mixture, N,N'-carbonyldiimidazole (2.73 g, 16.82 mmol) was added under ice-cooling, and the mixture was stirred for 42 hours after allowing the reaction temperature to the room temperature. Again under ice-cooling, O-methylhydroxylamine hydrochloride (7.02 g, 84.08 mmol) and triethylamine (11.7 mL, 84.08 mmol) were added to the mixture. After allowing the reaction temperature to the room temperature, the reaction mixture was stirred for 3 hours. The reaction mixture was partitioned with chloroform and saturated aqueous solution of sodium bicarbonate. The water layer was extracted with chloroform and the combined extract was washed with brine, followed by drying over MgSO₄ and distillation of the solvent under reduced pressure. The resultant residue was purified on a silica gel column chromatography to give a pale yellow solid which was then recrystallized from chloroform-ether to give the title compound as white crystals (4.52 g, 80%).
mp: 204-205°C

| Elemental Analysis for C₃₆H₃₁N₅O₄SF₂ | | | |
|---|---|---|---|
| | C(%) | H(%) | N(%) |
| Calcd. | 64.75; | 4.68; | 10.49 |
| Found | 64.61; | 4.67; | 10.31 |

¹H-NMR(300 MHz, CDCl₃) δ: 2.05 (3H, s), 3.57 (2H, s), 3.82 (3H, s), 3.90 (2H, s), 5.37 (2H, s), 6.92 (2H, d, J = 8.2 Hz), 7.16-7.31 (9H, m), 7.42-7.57 (5H, m), 7.63 (1H, s), 7.73 (2H, d, J = 8.8 Hz).
IR(KBr): 3338, 3064, 1717, 1669, 1628, 1591, 1531, 1470 cm⁻¹.

### Reference Example 8

### 3-(N-Benzyl-N-methylaminomethyl)-4,7-dihydro-5-isobutyryl-7-(2,6-difluorobenzyl)-2-[4-[(1-hydroxycyclopropyl)carbonylamino]phenyl]-4-oxothieno[2,3-b]pyridine

Diisopropylethylamine (0.52 g, 4 mmol) and 2-hydroxycyclopropanecarboxylic acid (0.204 g, 2 mmol) was added to a solution of 2-(4-aminophenyl)-7-(2,6-difluorobenzyl)-4,7-dihydro-5-isobutyryl-3-(N-benzyl-N-methylaminomethyl)-4-oxothieno[2,3-b]pyridine (0.57 g, 1.0 mmol) in dichloromethane (10 mL) and the mixture was stirred under ice-cooling. To this mixture was added benzotriazol-1-yloxytris(dimethylamino)phosphonium hexafluorophosphate (BOP reagent) (1.76 g, 4 mmol). The solution was stirred for 1 hour under ice-cooling, followed by stirring at room temperature for 4 days. After evaporating the reaction mixture under reduced pressure, the resultant residue was partitioned with water (50 mL) and chloroform (50 mL). The water layer was extracted again with chloroform (10 mL). The combined extract was washed with brine, and after drying over MgSO₄, the solvent was distilled off under reduced pressure. The resultant residue was purified on silica gel column chromatography, and recrystallized from ether to give yellow powder crystals (0.27 g, 41%).
¹H-NMR(300 MHz, CDCl₃) δ: 1.16-1.20 (2H, m), 1.18 (6H, d), 1.48-1.51 (2H, m), 2.09 (3H, s), 3.64 (2H, s), 3.95 (1H, br s), 4.14 (2H, s), 4.12-4.19 (1H, m), 5.20 (2H, s), 6.99 (2H, t), 7.10-7.25 (5H, m), 7.34-7.46 (1H, m), 7.57 (2H, d), 7.70 (2H, d), 8.21 (1H, s), 8.82 (1H, s).

### Example 1

### 5-(N-Benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione (hereinafter to be abbreviated as Compound A)-comprising solid preparation

The pharmaceutical preparations having the formulations shown in Table 1 were produced as follows.
(1) Hydroxypropyl methylcellulose 2910 (TC-5) (400.5 g) was dissolved in purified water (4050 g). Titanium oxide (45 g) and yellow ferric oxide (4.5 g) were dispersed in the obtained solution to give a coating agent.
(2) Compound A (730 g) obtained in Reference Example 7, lactose (2081 g), corn starch (365 g) and calcium carboxymethylcellulose (carmellose calcium, 182.5 g) were placed in a fluidized granulation dryer (manufactured by POWREX), pre-heated and mixed, and a 20% ethanol solution (2920 g), in which hydroxypropyl cellulose (146 g) had been dissolved, was sprayed to give granules. The production conditions in the granulation step are shown in Table 2.
(3) The granules (3144 g) obtained in the aforementioned (2) were sized with Power Mill (manufactured by Showakagakukikaikosakusho) to give sized granules. The obtained sized granules (2976 g), carmellose calcium (93 g) and magnesium stearate (31 g) were mixed in a tumbler mixer (manufactured by Showakagakukikaikosakusho) to give a powder mixture. This powder mixture (2900 g) was tableted with a tableting machine (manufactured by Kikusui Seisakusho Ltd.) to give uncoated tablets. The production conditions for sizing, mixing and tableting steps are shown in Table 2.
(4) The coating agent obtained in the aforementioned (1) was sprayed on the uncoated tablets obtained in the aforementioned (3) in a film-coating machine (manufactured by POWREX) to give 5,000 film-coated tablets comprising 100 mg of Compound A per tablet and having the formulation shown in Table 1. The production conditions for the film-coating step are shown in Table 2.

**Table 1**

| Formulation of tablet (composition per tablet): | | |
|---|---|---|
| composition | | amount added (mg) |
| 1) | compound A | 100.0 |
| 2) | lactose | 285.0 |
| 3) | corn starch | 50.0 |
| 4) | hydroxypropyl cellulose | 20.0 |
| 5) | carmellose calcium | 40.0 |
| 6) | magnesium stearate | 5.0 |
| | uncoated tablet | 500.0 |
| | | |

| (film components) | | |
|---|---|---|
| 7) | hydroxypropyl methylcellulose 2910 | 17.8 |
| 8) | titanium oxide | 2.0 |
| 9) | yellow ferric oxide | 0.2 |
| | total | 520.0 |

**[Table 2]**

| production conditions: | | |
|---|---|---|
| step | item | record |
| Granulation | Air supply temperature (°C) | 60-64 |
| | Air supply flow (m³/min) | 3.0-3.5 |
| | Liquid addition rate (g/min) | 66 |
| | Air exhaust temperature (°C) | 25-30 |
| | Product temperature at steady-state (°C) | 26 |
| | Pre-heating time (min) | 1 |
| | Drying time (min) | 6 |
| Sizing | Screen size (mm_{φ}) | 1.5 |
| | Rotation speed (rpm) | 3000 |
| Mixing | Rotation speed (rpm) | 30 |
| | Mixing time (min) | 3 |
| Tableting | Punch shape | 14 mm×8 mm (oval tablet) |
| | Tableting pressure (kN/punch) | 5.7 |
| | Rotation speed (rpm) | 30 |
| Film-coating | Air supply temperature (°C) | 70 |
| | Air supply flow (m³/min) | 4.5 |
| | Liquid addition rate (g/min) | 15 |
| | Pan rotation speed (rpm) | 9 |
| | Product temperature at steady-state (°C) | 45 |

### Example 2

### 3-(N-Benzyl-N-methylaminomethyl)-4,7-dihydro-5-isobutyryl-7-(2,6-difluorobenzyl)-2-[4-[(1-hydroxycyclopropyl)carbonylamino]phenyl]-4-oxothieno[2,3-b]pyridine (hereinafter to be abbreviated as Compound B)-comprising solid preparation

The pharmaceutical preparations having the formulations shown in Table 3 were produced as follows.
(1) Hydroxypropyl methylcellulose 2910 (TC-5) (405.8 g) was dissolved in purified water (4104 g). Titanium oxide (45.6 g) and yellow ferric oxide (4.56 g) were dispersed in the obtained solution to give a coating agent.
(2) Compound B (1100 g) obtained in Reference Example 8, lactose (2119 g), corn starch (418 g) and calcium carboxymethylcellulose (carmellose calcium, 209 g) were placed in a fluidized granulator dryer (manufactured by POWREX), pre-heated and mixed, and an aqueous solution (3344 g), in which hydroxypropyl cellulose (167.2 g) had been dissolved, was sprayed to give granules.
(3) The granules (3684 g) obtained in the aforementioned (2) were sized with Power Mill (manufactured by Showakagakukikaikosakusho) to give sized granule. The obtained sized granules (3539 g), carmellose calcium (110.6 g) and magnesium stearate (36.86 g) were mixed in a tumbler mixer (manufactured by Showakagakukikaikosakusho) to give a powder mixture. This powder mixture (3496 g) was tableted with a tableting machine (manufactured by Kikusui Seisakusho Ltd.) to give uncoated tablets.
(4) The coating agent obtained in the aforementioned (1) was sprayed on the uncoated tablets obtained in the aforementioned (3) in a film-coating machine (manufactured by POWREX) to give 7,000 film-coated tablets comprising 100 mg of Compound B per tablet and having the formulation shown in Table 3. The production conditions for each step are shown in Table 4.

**Table 3**

| Formulation of tablet (composition per tablet): | | |
|---|---|---|
| composition | | amount added (mg) |
| 1) | compound B | 100.0 |
| 2) | lactose | 192.6 |
| 3) | corn starch | 38.0 |
| 4) | hydroxypropyl cellulose | 15.2 |
| 5) | carmellose calcium | 30.4 |
| 6) | magnesium stearate | 3.8 |
| | uncoated tablet | 380.0 |
| | | |

| (film components) | | |
|---|---|---|
| 7) | hydroxypropyl methylcellulose 2910 | 10.68 |
| 8) | titanium oxide | 1.2 |
| 9 | yellow ferric oxide | 0.12 |
| | total | 392.0 |

**Table 4**

| production conditions: | | |
|---|---|---|
| step | item | record |
| Granulation | Air supply temperature (°C) | 67-70 |
| | Air supply flow (m³/min) | 3.5-3.8 |
| | Liquid addition rate (g/min) | 70 |
| | Air exhaust temperature (°C) | 27-28 |
| | Product temperature at steady-state (°C) | 27.5 |
| | Pre-heating time (min) | 1 |
| | Drying time (min) | 8 |
| Sizing | Screen size (mm_{φ}) | 1.5 |
| | Rotation speed (rpm) | 3000 |
| Mixing | Rotation speed (rpm) | 30 |
| | Mixing time (min) | 3 |
| Tableting | Punch shape | 13 mm×8 mm (oval tablet) |
| | Tableting pressure (kN/punch) | 5.8 |
| | Rotation speed (rpm) | 25-30 |
| Film-coating | Air supply temperature (°C) | 70 |
| | Air supply flow (m³/min) | 4.5 |
| | Liquid addition rate (g/min) | 15 |
| | Pan rotation speed (rpm) | 9 |
| | Product temperature at steady-state (°C) | 46 |

### Example 3

Compound A (100 g), lactose (116 g) and corn starch (60 g) were placed in a fluidized granulation dryer (manufactured by POWREX), pre-heated and mixed, and an aqueous solution (150 g), in which hydroxypropyl cellulose (9 g) had been dissolved, was sprayed to give granules having the formulation shown in Table 5.

### Example 4

Compound A (1000 g), lactose (1070 g) and corn starch (600 g) were placed in a fluidized granulation dryer (manufactured by POWREX), pre-heated and mixed, and an aqueous solution (2500 g), in which hydroxypropyl cellulose (150 g) had been dissolved, was sprayed to give granules having the formulation shown in Table 5.

### Example 5

Compound A (200 g), lactose (560 g), corn starch (100 g) and calcium carboxymethylcellulose (carmellose calcium) (50 g) were placed in a fluidized granulation dryer (manufactured by POWREX), pre-heated and mixed, and an aqueous solution (834 g), in which hydroxypropyl cellulose (50 g) had been dissolved, was sprayed to give granules having the formulation shown in Table 5.

**Table 5**

| Formulation of granule (composition per tablet, unit mg) | | | | | |
|---|---|---|---|---|---|
| | composition | Example 1 | Example 3 | Example 4 | Example 5 |
| 1) | compound A | 100.0 | 100.0 | 100.0 | 100.0 |
| 2) | lactose | 285.0 | 116.0 | 107.0 | 280.0 |
| 3) | corn starch | 50.0 | 60.0 | 60.0 | 50.0 |
| 4) | carmellose calcium | 25.0 | - | - | 25.0 |
| 5) | hydroxypropyl cellulose | 20.0 | 9.0 | 15.0 | 25.0 |
| | granules | 480.0 | 285.0 | 282.0 | 480.0 |

### Experimental Example 1

Evaluation of productivity (granulability) of the granules of Example 1-(2) and Examples 3-5 are shown in Table 6.

In Examples 3-5, granulation was insufficient and the obtained granules showed poor handling property. Tableting was difficult but production of pharmaceutical preparation was possible. In contrast, the granules obtained in Example 1-(2) were sufficiently granulated and no problem was observed in the tableting step.

**Table 6**

| | Example 1-(2) | Example 3 | Example 4 | Example 5 |
|---|---|---|---|---|
| Granulability | ○ | × | × | × |
| ○: Granules were obtained. | | | | |
| ×: Due to insufficient granulation, the content of fine powder in granules was high. | | | | |

### Experimental Example 2

Changing the production scale in Example 1 from 5,000 tablets to 600 tablets, uncoated tablets and film-coated tablets, comprising 25 mg of compound A per tablet, were obtained according to the method described in Example 1 and subjected to photo-stability test, the results of which are shown in Table 7.

Changing the production scale in Example 2 from 7,000 tablets to 580 tablets, uncoated tablets and film-coated tablets, both comprising 25 mg of compound B per tablet, were obtained according to the method described in Example 2 and subjected to photo-stability test, the results of which are shown in Table 8.

In both cases, the surface of the uncoated tablets exposed to light turned yellow, and the tablets showed a remarkable increase of related substances, as compared to the control (light-block tablets). In contrast, the film-coated tablets did not show changes in the appearance upon irradiation of light, and related substances almost did not increase as compared to the control (light-block tablets).

**Table 7**

| | Test conditions | Appearance | Residual ratio (%) | Related substance (%) |
|---|---|---|---|---|
| Uncoated tablet | Xe irradiation (1200000 lux·hr) | Turned yellow | 96.9 | 2.01 |
| (compound A tablet) | Control (light-block) | no change | 98.1 | 1.57 |
| Film-coated tablet | Xe irradiation (1200000 lux·hr) | no change | 101.1 | 1.53 |
| (compound A tablet) | Control (light-block) | no change | 101.8 | 1.45 |

**Table 8**

| | Test conditions | Appearance | Residual ratio (%) | Related substance (%) |
|---|---|---|---|---|
| Uncoated tablet | Xe irradiation (1200000 lux·hr) | Turned yellow | 98.8 | 1.18 |
| (compound B tablet) | Control (light-block) | no change | 100.4 | 0.69 |
| Film-coated tablet | Xe irradiation (1200000 lux·hr) | no change | 101.0 | 0.80 |
| (compound B tablet) | Control (light-block) | no change | 98.8 | 0.65 |

### Example 6

Compound A (600 g), lactose (1710 g), corn starch (300 g) and calcium carboxymethylcellulose (carmellose calcium) (150 g) were placed in a fluidized granulation dryer (manufactured by POWREX), pre-heated and mixed, and a 20% ethanol solution (2400 g), in which hydroxypropyl cellulose (120 g) had been dissolved, was sprayed to give granules. The obtained granules (2500 g) were sized using Power Mill (manufactured by Showakagakukikaikosakusho) to give sized granule. The obtained sized granule (1920 g), carmellose calcium (60 g) and magnesium stearate (20 g) were admixed in a tumbler mixer (manufactured by Showakagakukikaikosakusho) to give a powder mixture, which was tableted using a tableting machine (manufactured by Shimadzu Corporation) to give uncoated tablets having the formulation shown in Table 9. The tableting conditions are shown in Table 10.

**Table 9**

| Formulation of tablet (composition per tablet): | | |
|---|---|---|
| composition | | amount added (mg) |
| 1) | compound A | 100.0 |
| 2) | lactose | 285.0 |
| 3) | corn starch | 50.0 |
| 4) | hydroxypropyl cellulose | 20.0 |
| 5) | carmellose calcium | 40.0 |
| 6) | magnesium stearate | 5.0 |
| | uncoated tablet | 500.0 |

**Table 10**

| Tableting conditions: | | |
|---|---|---|
| step | item | record |
| Tableting | Punch shape | 12 mm_{φ} (round tablet) |
| | Tableting pressure (kN/punch) | 7.3 |

### Experimental Example 3

The dissolution properties of the uncoated tablets obtained in Example 1-(3) and Example 6 were compared.

As a test solution, citrate buffer (pH 3.0) comprising Tween 80 in a proportion of 0.1% was used and the dissolution rate was measured according to Japan Pharmacopoeia, the Second Method (puddle method, 50 rpm).

The results are shown in Fig. 1. From this, it is clear that the dissolution rate of the oval tablet of Example 1-(3) is faster than that of the round tablet of Experimental Example 6.

### Example 7

### 1) 5 mg tablet

(1) Hydroxypropyl methylcellulose 2910 (TC-5) (445 g) was dissolved in purified water (4500 g). Titanium oxide (50 g) and yellow ferric oxide (5 g) were dispersed in the obtained solution to give a coating agent.
(2) Compound A (42 g) obtained in Reference Example 7, lactose (3192 g), corn starch (420 g) and calcium carboxymethylcellulose (carmellose calcium) (210 g) were placed in a fluidized granulation dryer (manufactured by POWREX), pre-heated and mixed, and a 20% ethanol solution (3360 g), in which hydroxypropyl cellulose (168 g) had been dissolved, was sprayed to give granules. The production conditions in the granulation step were the same as those in Table 2.
(3) The granules (3528 g) obtained in the aforementioned (2) were sized using Power Mill (manufactured by Showakagakukikaikosakusho) to give sized granule. The obtained sized granule (3168 g), carmellose calcium (99 g) and magnesium stearate (33 g) were admixed in a tumbler mixer (manufactured by Showakagakukikaikosakusho) to give a powder mixture. The powder mixture (3000 g) was tableted using a tableting machine (manufactured by Kikusui Seisakusho Ltd.) to give uncoated tablets. The production conditions in the sizing, mixing and tableting steps were the same as those in Table 2.
(4) The coating agent obtained in the aforementioned (1) was sprayed in a film-coating machine (manufactured by POWREX) on the uncoated tablets obtained in the aforementioned (3) to give 5,000 film-coated tablets comprising 5 mg of Compound A per tablet and having the formulation shown in Table 11. The production conditions in the film-coating step were the same as those in Table 2.

### 2) 50 mg tablet

(1) Hydroxypropyl methylcellulose 2910 (TC-5) (1780 g) was dissolved in purified water (18000 g). Titanium oxide (200 g) and yellow ferric oxide (20 g) were dispersed in the obtained solution to give a coating agent.
(2) Compound A (1500 g) obtained in Reference Example 7, lactose (10050 g), corn starch (1500 g) and calcium carboxymethylcellulose (carmellose calcium, 750 g) were placed in a fluidized granulation dryer (manufactured by POWREX), pre-heated and mixed, and a 20% ethanol solution (12000 g), in which hydroxypropyl cellulose (600 g) had been dissolved, was sprayed to give granules. The production conditions in the granulation step were the same as those in Table 2.
(3) The granules (12960 g) obtained in the aforementioned (2) were sized with Power Mill (manufactured by Showakagakukikaikosakusho) to give sized granule. The obtained sized granules (36000 g), carmellose calcium (1125 g) and magnesium stearate (375 g) were mixed in a tumbler mixer (manufactured by Showakagakukikaikosakusho) to give a powder mixture. This powder mixture (36500 g) was tableted with a tableting machine (manufactured by Kikusui Seisakusho Ltd.) to give uncoated tablets. The production conditions in the sizing, mixing and tableting steps were the same as those in Table 2.
(4) The coating agent obtained in the aforementioned (1) was sprayed in a film-coating machine (manufactured by POWREX) on the uncoated tablets obtained in the aforementioned (3) to give 66,000 film-coated tablets comprising 50 mg of Compound A per tablet and having the formulation shown in Table 11. The production conditions in the film-coating step were the same as those in Table 2.

**Table 11**

| Formulation of tablet (composition per tablet): | | | |
|---|---|---|---|
| | composition | amount added (mg) | |
| | | 5 mg tablet | 50 mg tablet |
| 1) | compound A | 5.0 | 50.0 |
| 2) | lactose | 380.0 | 335.0 |
| 3) | corn starch | 50.0 | 50.0 |
| 4) | hydroxypropyl cellulose | 20.0 | 20.0 |
| 5) | carmellose calcium | 40.0 | 40.0 |
| 6) | magnesium stearate | 5.0 | 5.0 |
| | uncoated tablet | 500.0 | 500.0 |
| | | | |

| (film components) | | | |
|---|---|---|---|
| 7) | hydroxypropyl methylcellulose 2910 | 17.8 | 17.8 |
| 8) | titanium oxide | 2.0 | 2.0 |
| 9) | yellow ferric oxide | 0.2 | 0.2 |
| | total | 520.0 | 520.0 |

### Example 8

(1) Compound A (8.4 g), lactose (3226 g), corn starch (420 g) and calcium carboxymethylcellulose (carmellose calcium, 210 g) were placed in a fluidized granulation dryer (manufactured by POWREX), pre-heated and mixed, and an aqueous solution (3.360 g), in which hydroxypropyl cellulose (168 g) had been dissolved, was sprayed to give granules. The production conditions in the granulation step are shown in Table 13.
(3) The granules (3528 g) obtained in the aforementioned (1) were sized with Power Mill (manufactured by Showakagakukikaikosakusho) to give sized granule. The obtained sized granules (3168 g), carmellose calcium (99 g) and magnesium stearate (33 g) were mixed in a tumbler mixer (manufactured by Showakagakukikaikosakusho) to give a powder mixture shown in Table 12. The production conditions in the sizing and mixing steps are shown in Table 13.
(3) The powder mixture obtained in the aforementioned (2) is tableted in a tableting machine (manufactured by Kikusui Seisakusho Ltd.) and a coating agent (coating agent obtained by mixing a solution of hydroxypropyl methylcellulose 2910 (TC-5) in purified water and a suspension of titanium oxide and yellow ferric oxide) is sprayed in a film-coating machine (manufactured by POWREX) on the obtained uncoated tablets, whereby film-coated tablets comprising 1 mg of Compound A per tablet and having the formulation shown in Table 12 can be obtained.

**Table 12**

| Formulation of tablet (composition per tablet): | | |
|---|---|---|
| composition | | amount added (mg) |
| 1) | compound A | 1.0 |
| 2) | lactose | 384.0 |
| 3) | corn starch | 50.0 |
| 4) | hydroxypropyl cellulose | 20.0 |
| 5) | carmellose calcium | 40.0 |
| 6) | magnesium stearate | 5.0 |
| | uncoated tablet | 500.0 |
| | | |

| (film components) | | |
|---|---|---|
| 7) | hydroxypropyl methylcellulose 2910 | 17.8 |
| 8) | titanium oxide | 2.0 |
| 9) | yellow ferric oxide | 0.2 |
| | total | 520.0 |

**Table 13**

| production conditions: | | |
|---|---|---|
| step | item | record |
| Granulation | Air supply temperature (°C) | 70-75 |
| | Air supply flow (m³/min) | 3.5 |
| | Liquid addition rate (g/min) | 68 |
| | Air exhaust temperature (°C) | 29-33 |
| | Product temperature at steady-state (°C) | 38 |
| | Pre-heating time (min) | 2 |
| | Drying time (min) | 3 |
| Sizing | Screen size (mm_{φ}) | 1.5 |
| | Rotation speed (rpm) | 3000 |
| Mixing | Rotation speed (rpm) | 30 |
| | Mixing time (min) | 3 |

### Example 9

Compound A (25 g), lactose (167.5 g), corn starch (25 g) and calcium carboxymethylcellulose (carmellose calcium, 12.5 g) were placed in a fluidized granulation dryer (manufactured by POWREX), pre-heated and mixed, and an aqueous solution (200 g), in which hydroxypropyl cellulose (10 g) had been dissolved, was sprayed to give granules shown in Table 14. The obtained granules underwent sufficient granulation and the tableting step was free of problems.

**Table 14**

| Formulation of granules (composition per tablet): | | |
|---|---|---|
| composition | | amount added (mg) |
| 1) | compound A | 50.0 |
| 2) | lactose | 335.0 |
| 3) | corn starch | 50.0 |
| 4) | carmellose calcium | 25.0 |
| 6) | magnesium stearate | 20.0 |
| | uncoated tablet | 480.0 |

### Example 10

(1) Compound A (700 g), lactose (2576 g), corn starch (420 g) and calcium carboxymethylcellulose (carmellose calcium, 210 g) were placed in a fluidized granulation dryer (manufactured by POWREX), pre-heated and mixed, and an aqueous solution (1974 g), in which hydroxypropyl cellulose (126 g) had been dissolved, was sprayed to give granules. The production conditions in the granulation step are shown in Table 16. The obtained granules underwent sufficient granulation and the tableting step was free of problems.
(2) The granules (3950 g) obtained in the aforementioned (1) were sized with Power Mill (manufactured by Showakagakukikaikosakusho) to give sized granules. The obtained sized granules (3168 g), carmellose calcium (99 g) and magnesium stearate (33 g) were mixed in a tumbler mixer (manufactured by Showakagakukikaikosakusho) to give a powder mixture. This powder mixture (1000 g) was tableted with a tableting machine (manufactured by Kikusui Seisakusho Ltd.) to give uncoated tablets shown in Table 15. The production conditions in the sizing, mixing and tableting steps are shown in Table 16.
(3) A coating agent (coating agent obtained by mixing a solution of hydroxypropyl methylcellulose 2910 (TC-5) in purified water and a suspension of titanium oxide and yellow ferric oxide) is sprayed in a film-coating machine (manufactured by POWREX) on the uncoated tablets obtained in the aforementioned (2), whereby film-coated tablets comprising 50 mg of Compound A per tablet and having the formulation shown in Table 15 can be obtained.

**Table 15**

| Formulation of tablet (composition per tablet): | | |
|---|---|---|
| composition | | amount added (mg) |
| 1) | compound A | 50.0 |
| 2) | lactose | 184.0 |
| 3) | corn starch | 30.0 |
| 4) | hydroxypropyl cellulose | 9.0 |
| 5) | carmellose calcium | 24.0 |
| 6) | magnesium stearate | 3.0 |
| | uncoated tablet | 300.0 |

| (film components) | | |
|---|---|---|
| 7) | hydroxypropyl methylcellulose 2910 | 10.68 |
| 8) | titanium oxide | 1.2 |
| 9) | yellow ferric oxide | 0.12 |
| | total | 312.0 |

**Table 16**

| production conditions: | | |
|---|---|---|
| step | item | record |
| Granulation | Air supply temperature (°C) | 65-70 |
| | Air supply flow (m³/min) | 2.5-3.5 |
| | Liquid addition rate (g/min) | 68 |
| | Air exhaust temperature (°C) | 25-30 |
| | Product temperature at steady-state (°C) | 27 |
| | Pre-heating time (min) | 3 |
| | Drying time (min) | 10 |
| Sizing Mixing Tableting | Screen size (mm_{φ}) | 1.5 |
| | Rotation speed (rpm) | 3000 |
| | Rotation speed (rpm) | 20 |
| | Mixing time (min) | 3 |
| | Punch shape | 9.5 mm (round tablet) |
| | Tableting pressure (kN/punch) | 3.0 |
| | Rotation speed (rpm) | 25 |

### Example 11

### 1) 5 mg tablet

(1) Compound A (210 g), lactose (3066 g), corn starch (420 g) and calcium carboxymethylcellulose (carmellose calcium) (210 g) are placed in a fluidized granulation dryer (manufactured by POWREX), pre-heated and mixed, and an aqueous solution (1974 g), in which hydroxypropyl cellulose (126 g) has been dissolved, is sprayed to give granules.
(2) The granules (3528 g) obtained in the aforementioned (1) are sized with Power Mill (manufactured by Showakagakukikaikosakusho) to give sized granule. The obtained sized granules (3168 g), carmellose calcium (99 g) and magnesium stearate (33 g) are mixed in a tumbler mixer (manufactured by Showakagakukikaikosakusho) to give a powder mixture. This powder mixture (3000 g) is tableted with a tableting machine (manufactured by Kikusui Seisakusho Ltd.) to give uncoated tablets shown in Table 17.
(3) A coating agent (coating agent obtained by mixing a solution of hydroxypropyl methylcellulose 2910 (TC-5) in purified water and a suspension of titanium oxide and yellow ferric oxide) is sprayed in a film-coating machine (manufactured by POWREX) on the obtained uncoated tablets in the aforementioned (2), whereby film-coated tablets comprising 5 mg of Compound A per tablet and having the formulation shown in Table 17 can be obtained.

### 2) 25 mg tablet

(1) Compound A (420 g), lactose (2856 g), corn starch (420 g) and calcium carboxymethylcellulose (carmellose calcium) (210 g) are placed in a fluidized granulation dryer (manufactured by POWREX), pre-heated and mixed, and an aqueous solution (1974 g), in which hydroxypropyl cellulose (126 g) has been dissolved, is sprayed to give granules.
(2) The granules (3528 g) obtained in the aforementioned (1) are sized with Power Mill (manufactured by Showakagakukikaikosakusho) to give sized granule. The obtained sized granules (3168 g), carmellose calcium (99 g) and magnesium stearate (33 g) are mixed in a tumbler mixer (manufactured by Showakagakukikaikosakusho) to give a powder mixture. This powder mixture (3000 g) is tableted with a tableting machine (manufactured by Kikusui Seisakusho Ltd.) to give uncoated tablets shown in Table 17.
(3) A coating agent (coating agent obtained by mixing a solution of hydroxypropyl methylcellulose 2910 (TC-5.) in purified water and a suspension of titanium oxide and yellow ferric oxide) is sprayed in a film-coating machine (manufactured by POWREX) on the uncoated tablets obtained in the aforementioned (2), whereby film-coated tablets comprising 25 mg of Compound A per tablet and having the formulation shown in Table 17 can be obtained.

**Table 17**

| Formulation of tablet (composition per tablet): | | | |
|---|---|---|---|
| composition | | amount added (mg) | |
| | | 5 mg tablet | 50 mg tablet |
| 1) | compound A | 5.0 | 25.0 |
| 2) | lactose | 73.0 | 170.0 |
| 3) | corn starch | 10.0 | 25.0 |
| 4) | hydroxypropyl cellulose | 3.0 | 7.5 |
| 5) | carmellose calcium | 8.0 | 20.0 |
| 6) | magnesium stearate | 5.0 | 2.5 |
| | uncoated tablet | 100.0 | 250.0 |
| | | | |

| (film components) | | | |
|---|---|---|---|
| 7) | hydroxypropyl methylcellulose 2910 | 3.56 | 8.9 |
| 8) | titanium oxide | 0.4 | 1.0 |
| 9) | yellow ferric oxide | 0.04 | 0.1 |
| | total | 104.0 | 260.0 |

### Example 12

(1) Hydroxypropyl methylcellulose 2910 (TC-5) (405.8 g) was dissolved in purified water (4104 g). Titanium oxide (45.6 g) and yellow ferric oxide (4.56 g) were dispersed in the obtained solution to give a coating agent.
(2) Compound B (55 g) obtained in Reference Example 8, lactose (3163 g), corn starch (418 g) and calcium carboxymethylcellulose (carmellose calcium, 209 g) were placed in a fluidized granulation dryer (manufactured by POWREX), pre-heated and mixed, and an aqueous solution (3344 g), in which hydroxypropyl cellulose (167.2 g) had been dissolved, was sprayed to give granules.
(3) The granules (3539 g) obtained in the aforementioned (2) were sized with Power Mill (manufactured by Showakagakukikaikosakusho) to give sized granule. The obtained sized granules (3101 g), carmellose calcium (96.9 g) and magnesium stearate (32.3 g) were mixed in a tumbler mixer (manufactured by Showakagakukikaikosakusho) to give a powder mixture. This powder mixture (3116 g) was tableted with a tableting machine (manufactured by Kikusui Seisakusho Ltd.) to give uncoated tablets.
(4) The coating agent obtained in the aforementioned (1) was sprayed in a film-coating machine (manufactured by POWREX) on the uncoated tablets obtained in the aforementioned (3) to give 6,580 film-coated tablets comprising 5 mg of Compound B per tablet and having the formulation shown in Table 18. The production conditions in each step were the same as those in Table 4.

**Table 18**

| Formulation of tablet (composition per tablet): | | |
|---|---|---|
| composition | | amount added (mg) |
| 1) | compound B | 5.0 |
| 2) | lactose | 287.6 |
| 3) | corn starch | 38.0 |
| 4) | hydroxypropyl cellulose | 15.2 |
| 5) | carmellose calcium | 30.4 |
| 6) | magnesium stearate | 3.8 |
| | uncoated tablet | 380.0 |

| (film components) | | |
|---|---|---|
| 7) | hydroxypropyl methylcellulose 2910 | 10.68 |
| 8) | titanium oxide | 1.2 |
| 9) | yellow ferric oxide | 0.12 |
| | total | 392.0 |

### Industrial Applicability

By mixing a physiologically active substance, a cellulose-type disintegrant and a solution comprising a binder according to the present invention, granules having a pre-determined particle size and having improved granulability can be adjusted easily.

In addition, a solid preparation comprising the granule of the present invention, particularly a solid preparation comprising the granule of the present invention, a cellulose-type disintegrant and a stearic acid-type lubricant can comprise the physiologically active substance in a large content, and shows superior disintegration property and superior dissolution of the physiologically active substance. Particularly when a film-coated tablet is prepared, stable long-term preservation in a non-shading state is afforded.

This application is based on a patent application No. 289345/2000 filed in Japan, the contents of which are hereby incorporated by reference.

## Claims

1. A granule comprising a physiologically active substance and a cellulose-type disintegrant.

2. The granule of claim 1, wherein the cellulose-type disintegrant is a compound selected from calcium carboxymethylcellulose, carboxymethylcellulose, croscarmellose sodium and low substituted hydroxypropyl cellulose or comprises two or more compounds therefrom in combination.

3. The granule of claim 1, wherein the cellulose-type disintegrant is calcium carboxymethylcellulose.

4. The granule of claim 1, wherein the physiologically active substance is slightly soluble in water.

5. The granule of claim 1, wherein the physiologically active substance is water-repellent.

6. The granule of claim 1, wherein the physiologically active substance is slightly soluble in water and water-repellent.

7. The granule of claim 1, wherein the physiologically active substance has a contact angle of not less than 80 degrees.

8. The granule of claim 1, wherein the physiologically active substance is a compound having a partial structure represented by the formula: 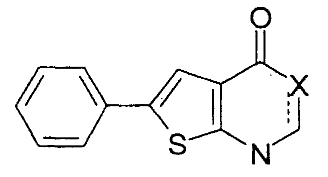 wherein X represents a carbon atom or a nitrogen atom, and
- - - represents a single bond or a double bond or a salt thereof.

9. The granule of claim 8, wherein the compound having a partial structure represented by the formula: 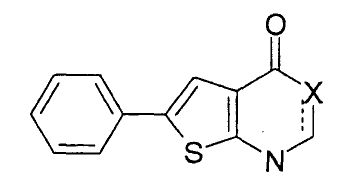 wherein X represents a carbon atom or a nitrogen atom, and
- - - represents a single bond or a double bond or a salt thereof is a compound represented by the formula: 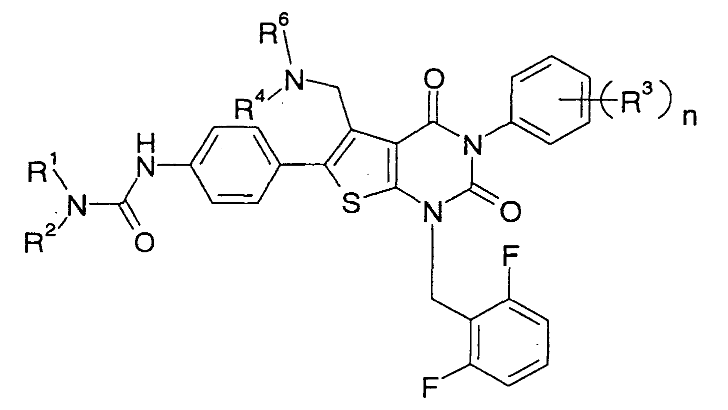 wherein R¹ and R² each represent a hydrogen atom, a hydroxy group, a C₁₋₄ alkoxy group, a C₁₋₄ alkoxy-carbonyl group or a C₁₋₄ alkyl group which may have a substituent,
R³ represents a hydrogen atom, a halogen atom, a hydroxy group or a C₁₋₄ alkoxy group which may have a substituent, or
adjacent two R³s may be linked to form a C₁₋₄ alkylenedioxy group;
R⁴ represents a hydrogen atom or a C₁₋₄ alkyl group;
R⁶ represents a C₁₋₄ alkyl group which may have a substituent or a group represented by the formula: 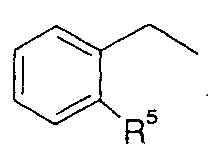 wherein R⁵ represents a hydrogen atom or R⁴ and R⁵ may be linked to form a heterocycle; and
n represents an integer of 0 to 5 or a salt thereof.

10. The granule of claim 8, wherein the compound having a partial structure represented by the formula: 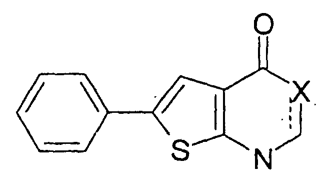 wherein X represents a carbon atom or a nitrogen atom, and
- - - represents a single bond or a double bond or a salt thereof is 5-(N-benzyl-N-methylaminomethyl)-1-(2,6-difluorobenzyl)-6-[4-(3-methoxyureido)phenyl]-3-phenylthieno [2,3-d]pyrimidine-2,4(1H,3H)-dione or a salt thereof.

11. The granule of claim 8, wherein the compound having a partial structure represented by the formula: 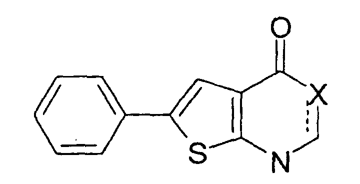 wherein X represents a carbon atom or a nitrogen atom, and
- - - represents a single bond or a double bond or a salt thereof is a compound represented by the formula: 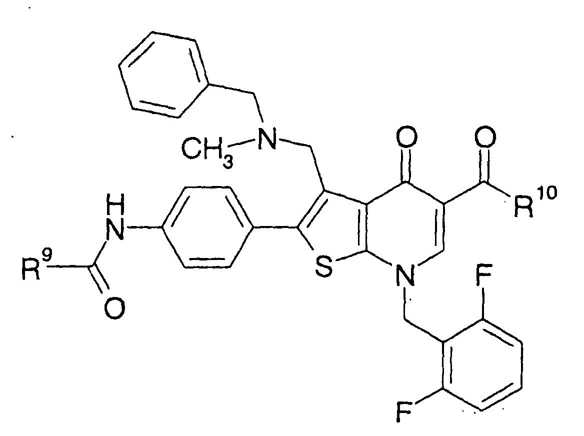 wherein R⁹ represents an optionally substituted C₁₋₇ alkyl group, an optionally substituted C₃₋₇ cycloalkyl group, an optionally substituted C₁₋₆ alkoxyamino group or an optionally substituted hydroxyamino group, and
R¹⁰ represents an optionally substituted C₁₋₇ alkyl group or an optionally substituted phenyl group, respectively; or
when R⁹ is an unsubstituted C₁₋₇ alkyl group, R¹⁰ represents a substituted C₁₋₇ alkyl group or a substituted phenyl or a salt thereof.

12. The granule of claim 8, wherein the compound having a partial structure represented by the formula: 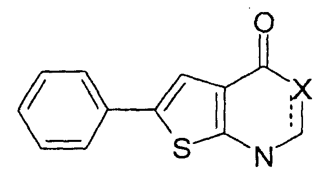 wherein X represents a carbon atom or a nitrogen atom, and
- - - represents a single bond or a double bond or a salt thereof is 3-(N-benzyl-N-methylaminomethyl)-4,7-dihydro-5-isobutyryl-7-(2,6-difluorobenzyl)-2-[4-[(1-hydroxycyclopropyl)carbonylamino]phenyl]-4-oxothieno[2,3-b]pyridine or a salt thereof.

13. The granule of claim 1, which is obtained by mixing and drying a physiologically active substance, a cellulose-type disintegrant and a solution comprising a binder.

14. The granule of claim 13, wherein the binder is a compound selected from hydroxypropyl cellulose, pre-gelatinized starch, sucrose, gelatin, powdered acacia, methylcellulose, hydroxypropyl methylcellulose, polyvinylpyrrolidone, dextrin and pullulan or two or more compounds therefrom in combination.

15. The granule of claim 13, wherein the binder is hydroxypropyl cellulose.

16. The granule of claim 13, wherein the solution comprising a binder is an alcohol solution.

17. The granule of claim 16, wherein the alcohol has 1 to 3 carbon atoms.

18. The granule of claim 16, wherein the alcohol is ethanol.

19. The granule of claim 1, which is used for tableting.

20. A production method of granule, which comprises mixing and drying a physiologically active substance, a cellulose-type disintegrant and a solution comprising a binder.

21. A solid preparation comprising the granule of claim 1 to 18.

22. The solid preparation of claim 21, comprising the granule of claim 1 to 18, a cellulose-type disintegrant and a stearic acid-type lubricant.

23. The solid preparation of claim 22, wherein the cellulose-type disintegrant is a compound selected from calcium carboxymethylcellulose, carboxymethylcellulose, croscarmellose sodium and low substituted hydroxypropyl cellulose or comprises two or more compounds therefrom in combination.

24. The solid preparation of claim 22, wherein the cellulose-type disintegrant is calcium carboxymethylcellulose.

25. The solid preparation of claim 22, wherein the stearic acid-type lubricant is magnesium stearate.

26. The solid preparation of claim 21, which is a film-coated tablet.

27. The solid preparation of claim 21, which is an oval tablet.

28. The solid preparation of claim 21, which is a capsule.

29. The solid preparation of claim 21, which is a granule or fine granule.

30. A granule comprising a compound represented by the formula: 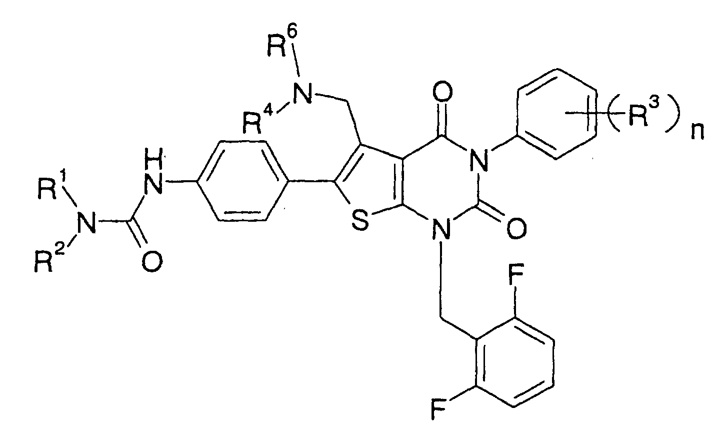 wherein R¹ and R² each represent a hydrogen atom, a hydroxy group, a C₁₋₄ alkoxy group, a C₁₋₄ alkoxy-carbonyl group or a C₁₋₄ alkyl group which may have a substituent,
R³ represents a hydrogen atom, a halogen atom, a hydroxy group or a C₁₋₄ alkoxy group which may have a substituent, or
adjacent two R³s may be linked to form a C₁₋₄ alkylenedioxy group;
R⁴ represents a hydrogen atom or a C₁₋₄ alkyl group;
R⁶ represents a C₁₋₄ alkyl group which may have a substituent or a group represented by the formula: 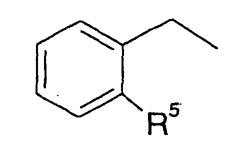 wherein R⁵ represents a hydrogen atom or R⁴ and R⁵ may be linked to form a heterocycle; and
n represents an integer of 0 to 5 or a salt thereof.

31. The granule of claim 30, which comprises 5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione or a salt thereof.

32. The granule of claim 30, which is obtained by mixing and drying a compound represented by the formula: 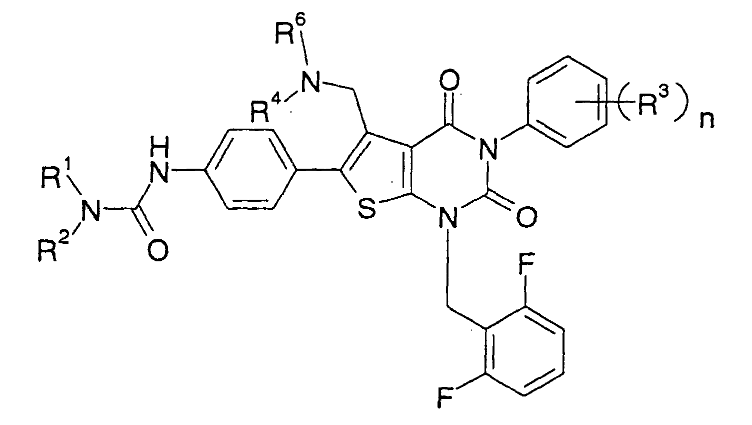 wherein R¹ and R² each represent a hydrogen atom, a hydroxy group, a C₁₋₄ alkoxy group, a C₁₋₄ alkoxy-carbonyl group or a C₁₋₄ alkyl group which may have a substituent,
R³ represents a hydrogen atom, a halogen atom, a hydroxy group or a C₁₋₄ alkoxy group which may have a substituent, or
adjacent two R³s may be linked to form a C₁₋₄ alkylenedioxy group;
R⁴ represents a hydrogen atom or a C₁₋₄ alkyl group;
R⁶ represents a C₁₋₄ alkyl group which may have a substituent or a group represented by the formula: 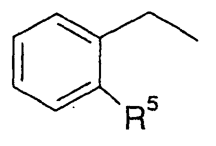 wherein R⁵ represents a hydrogen atom or R⁴ and R⁵ may be linked to form a heterocycle; and
n represents an integer of 0 to 5 or a salt thereof and a solution comprising a binder.

33. The granule of claim 32, wherein the binder is a compound selected from hydroxypropyl cellulose, pre-gelatinized starch, sucrose, gelatin, powdered acacia, methylcellulose, hydroxypropyl methylcellulose, polyvinylpyrrolidone, dextrin and pullulan or comprises two or more compounds in combination.

34. The granule of claim 32, wherein the solution comprising a binder is an alcohol solution.

35. The granule of claim 34, wherein the alcohol has 1 to 3 carbon atoms.

36. The granule of claim 34, wherein the alcohol is ethanol.

37. The granule of claim 30, which is used for tableting.

38. A production method of a granule, which comprises mixing and drying a compound represented by the formula: 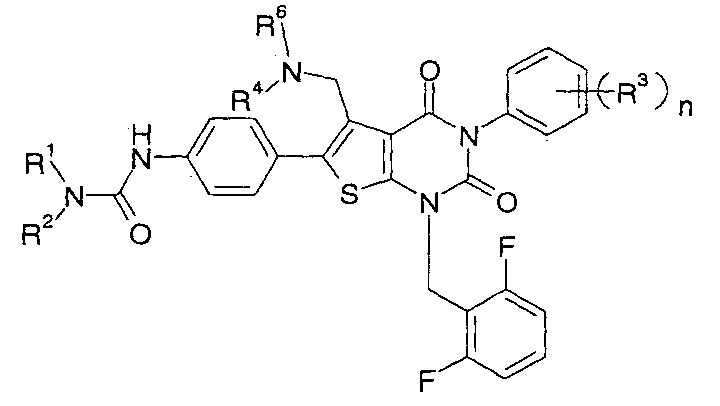 wherein R¹ and R² each represent a hydrogen atom, a hydroxy group, a C₁₋₄ alkoxy group, a C₁₋₄ alkoxy-carbonyl group or a C₁₋₄ alkyl group which may have a substituent,
R³ represents a hydrogen atom, a halogen atom, a hydroxy group or a C₁₋₄ alkoxy group which may have a substituent, or adjacent two R³s may be linked to form a C₁₋₄ alkylenedioxy group;
R⁴ represents a hydrogen atom or a C₁₋₄ alkyl group;
R⁶ represents a C₁₋₄ alkyl group which may have a substituent or a group represented by the formula: 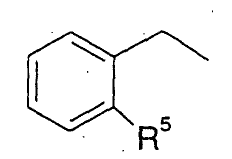 wherein R⁵ represents a hydrogen atom or R⁴ and R⁵ may be linked to form a heterocycle; and
n represents an integer of 0 to 5 or a salt thereof, and a solution comprising a binder.

39. A solid preparation comprising the granule of claims 30 to 36.

40. The solid preparation of claim 39, which comprises the granule of claims 30 to 36, a cellulose-type disintegrant and a stearic acid-type lubricant.

41. The solid preparation of claim 40, wherein the cellulose-type disintegrant is a compound selected from calcium carboxymethylcellulose, carboxymethylcellulose, croscarmellose sodium and low substituted hydroxypropyl cellulose or comprises two or more compounds thereof in combination.

42. The solid preparation of claim 40, wherein the cellulose-type disintegrant is calcium carboxymethylcellulose.

43. The solid preparation of claim 40, wherein the stearic acid-type lubricant is magnesium stearate.

44. The solid preparation of claim 39, which is a film-coated tablet.

45. The solid preparation of claim 39, which is an oval tablet.

46. The solid preparation of claim 39, which is a capsule.

47. The solid preparation of claim 39, which is a granule or a fine granule.

48. The solid preparation of claim 39, which is a gonadotropin releasing hormone antagonist.

49. The solid preparation of claim 39, which is an agent for the prophylaxis or treatment of sex hormone-dependent diseases.
